# EUROPEAN PATENT SPECIFICATION

(11) **EP 3 328 269 B1**
(45) Date of publication and mention of the grant of the patent: **30.09.2020**
(21) Application number: 16757976.2
(22) Date of filing: 02.08.2016
(51) Int. Cl.: A61B 5/00, A61B 5/107

(54) **A METHOD AND APPARATUS FOR MONITORING THE DEGREE OF OPENING OF THE CERVIX PRIOR TO THE ONSET OF LABOUR**
VERFAHREN UND VORRICHTUNG ZUR ÜBERWACHUNG DES ÖFFNUNGSGRADES DER ZERVIX VOR GEBURTSEINLEITUNG
PROCÉDÉ ET APPAREIL POUR SURVEILLER LE DEGRÉ D'OUVERTURE DU COL DE L'UTÉRUS AVANT LE DÉBUT DU TRAVAIL

(30) Priority: 31.07.2015 IE 20150220
(43) Date of publication of application: 06.06.2018
(73) Proprietor: Janisys Limited, Galway (IE)
(72) Inventor: O'DEA, John, Galway (IE)
(74) Representative: Gorman, Francis Fergus
(86) International application number: PCT/IE2016/000014
(87) International publication number: WO 2017/021947

(56) References cited:
- US-A1- 2007 239 197
- US-A1- 2010 094 328
- US-A1- 2010 113 939

## Description

The present invention relates to a method, apparatus and a balloon catheter for monitoring the degree of opening of a ripening cervix of a female mammal prior to the onset of labour.

US 2007/0239197 A1 and US 2010/0113939 A1 disclose devices which detect the opening of the cervix of a female mammal with the help of inflating balloons.

Cervical ripening, or opening of the cervix prior to the onset of labour in a normal pregnancy of a female human subject occurs naturally under hormonal control. Failure of the cervix to ripen at term increases the risk of delivery problems, including the need for caesarean section. Further consequences include prolonged hospital stays, increased medical costs, and an overall increase in maternal and foetal morbidity. In cases where a pregnancy has gone over term, which represents up to 30% of births, one common method of promoting ripening of the cervix is to utilise a balloon catheter, whereby a deflated balloon of the balloon catheter is placed in the uterus and inflated therein. The balloon is then urged downwardly against the opening of the cervix in order to simulate the effect of the head of a foetus against the cervix. The balloon, in general, when inflated is of diameter of the order of 4cm to 5cm. This procedure can take up to nine hours, and sometimes longer, and requires a nurse or midwife to check at regular intervals if the cervix has ripened, or opened to the extent indicative of the onset of labour. Such checks at regular intervals are carried out by urging the balloon downwardly in order to ascertain if it will pull through the cervix.

An alternative procedure to promote ripening of the cervix is to apply a suitable pharmaceutical substance into the opening of the cervix in order to chemically promote ripening of the cervix. However, this procedure also requires a nurse or midwife to carry out checks at regular intervals in order to monitor the progress of the ripening of the cervix.

Accordingly, these procedures tend to be labour intensive, and tend to be invasive. Additionally, by virtue of the fact that the checks must be carried out at regular intervals by a nurse or midwife, it is necessary for a subject to be hospitalised during the procedures. Since the procedures may take nine hours or more, considerable hospital bed space, which would otherwise be unnecessary, is required. Additionally, a subject's stay in hospital is also extended. Furthermore, by virtue of the invasive aspects of the checks which must be carried out, a subject, in general, is unable to sleep, and thus by the time labour commences, the subject may be in a poor state to deal with labour and birth of the baby.

There is therefore a need for a method and apparatus for monitoring the degree of opening of the cervix of a subject during ripening thereof prior to the onset of labour, which addresses at least some of the above discussed problems.

The present invention is directed towards providing a method, an apparatus and a balloon catheter for monitoring the degree of opening of the cervix of a subject during ripening thereof prior to the onset of labour.

According to the invention there is provided apparatus for monitoring the degree of opening of the cervix in a female mammal, the apparatus comprising an inflatable first balloon configured for locating in the cervix, a pressure sensing means for monitoring a pressure indicative of the pressure of a compressible inflating medium in the first balloon, and a means for determining the degree of opening of the cervix from the pressure of the inflating medium monitored by the pressure sensing means, and for producing a signal indicative of the degree of opening of the cervix.

In one aspect of the invention the first balloon is of length to extend through the length of the cervix.

In another aspect of the invention a retaining means is provided for retaining the inflating medium in the first balloon after it has been inflated in the cervix to a first predefined pressure. Preferably, the retaining means comprises a first valve. Advantageously, the retaining means is releasable for deflating the first balloon. Preferably, the retaining means is manually releasable. Ideally, the retaining means comprises a first non-return valve.

In another aspect of the invention the first balloon comprises a flexible material. Preferably, the first the first balloon comprises an elastic material. Advantageously, the first balloon comprises a polyurethane material.

In one aspect of the invention the first balloon is configured so that when the first balloon is located in the cervix and inflated therein to the first predefined pressure prior to commencement of ripening thereof, the first balloon expands with the opening extending through the cervix, as the opening extending through the cervix expands, and the first balloon remains in contact with the portion of the cervix defining the opening therethrough during ripening of the cervix.

Preferably, the first balloon is configured so that when inflated to the first predefined pressure, the first balloon has substantially no effect on the opening of the cervix.

Advantageously, the first balloon is configured to be inflated with a compressible inflating medium.

In another aspect of the invention the means for determining the degree of opening of the cervix from the pressure monitored by the pressure sensing means comprises a signal processing means configured to read a signal from the pressure sensing means and to determine the degree of opening of the cervix in response to the signal read from the pressure sensing means.

In another aspect of the invention a correlating means is provided, the correlating means being configured to correlate a plurality of reference pressure values of an inflating medium in a reference balloon located in a reference cervix with corresponding values of reference degrees of opening of the reference cervix.

Preferably, the reference pressure values correlated in the correlating means with the corresponding values of the reference degrees of opening of the reference cervix range from a first predefined reference pressure value corresponding to a value of a first predefined reference degree of opening of the reference cervix to a second predefined reference pressure value corresponding to a value of a second predefined reference degree of opening of the reference cervix.

Advantageously, the first predefined reference pressure value corresponds to the pressure value of the inflating medium in the reference balloon in the reference cervix when the value of the first predefined reference degree of opening of the reference cervix corresponds to the reference cervix being in a state prior to the commencement of ripening thereof.

In another aspect of the invention the second predefined reference pressure corresponds to the pressure of the inflating medium in the reference balloon in the reference cervix when the value of the second predefined reference degree of opening of the reference cervix corresponds to the reference cervix being fully ripened.

In one aspect of the invention the values of the reference degree of opening of the reference cervix in the correlating means comprises respective values of the volume of the opening through the reference cervix from the uterus to the vagina.

Preferably, the correlating means is electronically stored and is accessible to the signal processing means. Advantageously, the correlating means is stored in the signal processing means.

In another aspect of the invention the correlating means comprises a look-up table in which data indicative of the values of the degrees of opening of the reference cervix is tabulated against data indicative of corresponding pressure values indicative of the pressure of the inflating medium in the reference balloon in the reference cervix.

Preferably, the first balloon is substantially similar to the reference balloon.

In one aspect of the invention the signal processing means is configured to produce the signal indicative of the degree of opening of the cervix.

In another aspect of the invention the signal indicative of the degree of opening of the cervix is configured to be presented as a human sensory perceptible signal.

Preferably, the human sensory perceptible signal indicative of the degree of opening of the cervix comprises a visually perceptible signal.

Advantageously, the visually perceptible signal indicative of the degree of opening of the cervix comprises one of a representation of the degree of opening of the cervix and indicia indicative of the degree of opening of the cervix.

In one aspect of the invention the representation of the cervix comprises a graphical representation of the opening through the cervix from the uterus to the vagina superimposed on a graphical representation of the opening of a fully ripened cervix from the uterus to the vagina.

In another aspect of the invention the visually perceptible signal indicative of the degree of opening of the cervix comprises a display of a scale indicative of the degrees of opening of a reference cervix during ripening thereof from commencement of ripening to full ripening thereof, and a display of an indication on the scale of the current degree of opening of the cervix.

In a further aspect of the invention the visually perceptible representation of the degree of opening of the cervix comprises a display of a colour chart or a display of shades of a colour in a single colour chart, and an indication of a colour on the colour chart or an indication of a shade on the single colour chart indicative of the degree of opening of the cervix.

Preferably, the colours or the shades of colour in the colour chart change progressively, and the representation of the degree of opening of the cervix is superimposed on the colour chart so that as the cervix progressively opens, the visual representation of the cervix progressively extends into the progressively changing colours or shades of the colour of the colour chart.

Advantageously, the indicia indicative of the degree of opening of the cervix is indicative of volume of the opening extending through the cervix from the uterus to the vagina.

Advantageously, the indicia indicative of the degree of opening of the cervix comprises an alpha numeric indication of the degree of opening of the cervix.

In one aspect of the invention the signal processing means is configured to produce at least one of a ripened alert signal in response to the signal read from the pressure sensing means being indicative of the cervix being fully ripened, and an approaching ripening alert signal in response to the signal read from the pressure sensing means being indicative of the cervix approaching the fully ripened state, respectively.

Preferably, the signal processing means is configured to communicate with a display means for communicating the signals produced by the signal processing means to the display means.

Advantageously, the display means is configured to be responsive to the signal indicative of the degree of opening of the cervix from the signal processing means for displaying a visually perceptible signal indicative of the degree of opening of the cervix.

In another aspect of the invention the apparatus comprises the display means.

In another aspect of the invention a communicating means is provided for wirelessly transmitting the signals produced by the signal processing means. Preferably, the signals produced by the signal processing means are transmitted by the communicating means under the control of the signal processing means for reception by a smart mobile device.

Advantageously, the communicating means is configured to transmit the signals produced by the signal processing means in one of a Bluetooth protocol and a near field communications (NFC) protocol.

Ideally, the signals produced by the signal processing means are configured for reception by a smart mobile device paired with the communicating means and being one of Bluetooth enabled and NFC enabled.

In one aspect of the invention the signals produced by the signal processing means are configured for reception by a smart mobile device configured by an app.

Preferably, the signals produced by the signal processing means are configured for reception by a smart mobile phone configured by an app.

In another aspect of the invention the apparatus comprises a smart mobile device for receiving the signal indicative of the degree of opening of the cervix from the signal processing means, and the smart mobile device is configured by an app to display the one of the representation indicative of the degree of opening of the cervix and the indicia indicative of the degree of opening of the cervix.

Preferably, the smart mobile device is configured by the app to receive and interpret the signal produced by the signal processing means indicative of the degree of opening of the cervix.

In one aspect of the invention the smart mobile device is configured by the app to produce a first preselected audible signal in response to the ripened alert signal.

In another aspect of the invention the smart mobile device is configured by the app to produce a second preselected audible signal in response to the approaching ripening signal.

Preferably, an alerting means is configured for producing a human sensory perceptible alert signal in response to the one of the ripened alert signal and the approaching ripening alert signal.

Advantageously, each human sensory perceptible alert signal produced by the alerting means comprises one or more of a visually perceptible alert signal, an aurally perceptible alert signal, and a tactilely perceptible signal.

In one aspect of the invention the alerting means comprises a vibrator for producing the tactilely perceptible alert signal. Preferably, the vibrator is configured for locating in the vagina of the female mammal.

In another aspect of the invention the alerting means comprises a buzzer element for producing the aurally perceptible alert signal.

Preferably, the buzzer element is configured for one of wearing by the female mammal and locating in the vagina of the female mammal.

Advantageously, the buzzer element and the vibrator are configured as a single buzzer/vibrator unit.

Preferably, the pressure sensing means produces an electrical signal indicative of the monitored pressure.

Advantageously, the pressure sensing means communicates with the signal processing means by one of hardwiring connection and a wireless connection.

In one aspect of the invention the signal processing means and the communicating means are housed in a housing to be one of worn by the female mammal and located in the vagina of the female mammal.

In another aspect of the invention the buzzer element is housed in the housing. Preferably, the vibrator is housed in the housing. Advantageously, the pressure sensing means is housed in the housing.

In another aspect of the invention the pressure sensing means is one of located within the first balloon, and externally of the first balloon.

In another aspect of the invention the pressure sensing means is configured for locating in one of the vagina of the female mammal and externally of the vagina of the female mammal.

Preferably, the signal processing means comprises a microprocessor.

Advantageously, the pressure sensing means comprises a pressure sensor.

In another aspect of the invention a distal portion of the first balloon is configured to extend into the uterus of the female mammal, and to engage the cervix adjacent the uterus. Preferably, a proximal end of the first balloon is configured to extend into the vagina, and to engage a portion of the cervix adjacent the vagina.

Advantageously, the distal and proximal portions of the first balloon are configured to co-operate such that the engagement action of the proximal portion of the first balloon with the portion of the cervix adjacent the vagina acts to urge the distal portion of the first balloon into engagement with the cervix adjacent the uterus, so that the distal portion of the balloon simulates the pressure of the head of a foetus on the cervix adjacent the uterus.

In one aspect of the invention the first balloon comprises a first balloon of a balloon catheter.

In another aspect of the invention the first balloon is located on a catheter of the balloon catheter towards the distal end of the catheter.

In one aspect of the invention a first lumen extends through the catheter of the balloon catheter for accommodating the inflating medium to the first balloon.

In another aspect of the invention the pressure sensing means communicates with the first lumen externally of the first balloon, and is configured to monitor the pressure of the inflating medium in the first lumen which is indicative of the pressure of the inflating medium in the first balloon.

In another aspect of the invention a second balloon is located on the catheter distally of the first balloon and adjacent thereto, the second balloon being configured for locating in the uterus when the first balloon is located in the cervix, and the second balloon is configured to bear on the cervix adjacent the uterus.

Preferably, the first balloon comprises a proximal portion configured to extend into the vagina adjacent the cervix, and to bear on the cervix for urging the second balloon into engagement with the cervix adjacent the uterus.

In another aspect of the invention a means is provided for applying a force to the second balloon in a direction outwardly relative to the vagina for urging the second balloon into engagement with the cervix adjacent the uterus. Preferably, the means for applying the force comprises an urging means for applying the outward force to the second balloon.

In one aspect of the invention the urging means is secured to one of the catheter and the second balloon.

Preferably, the urging means comprises a tensioning means.

Advantageously, the urging means comprises a ligature.

Preferably, the urging means comprises one of an elastic and a resilient material.

In one aspect of the invention the urging means is configured to progressively urge the second balloon progressively through the opening extending through the cervix as the cervix is progressively ripening.

In another aspect of the invention the urging means is configured to urge the second balloon to exit the cervix on the cervix reaching its fully ripened state.

In another aspect of the invention the urging means is configured to apply a substantially constant force to the second balloon as the cervix progressively ripens.

Preferably, the second balloon is configured to be inflated with an inflating medium which is the same or different to the inflating medium with which the first balloon is configured to be inflated.

Advantageously, the second balloon is configured to be inflated with one of a compressible inflating medium and an incompressible inflating medium.

In one aspect of the invention the catheter of the balloon catheter is of length so that the proximal end of the catheter terminates adjacent the entrance to the vagina.

Preferably, the first lumen terminates in the first valve adjacent the proximal end of the catheter.

In one aspect of the invention one of the proximal end of the catheter of the balloon catheter and the first valve terminates in a coupling means configured for coupling the first balloon to a source of the inflating medium.

Preferably, the coupling means is configured for coupling a syringe to one of the first lumen and the first valve.

In another aspect of the invention the housing within which the signal processing means and the communicating means is located is mounted on the catheter of the balloon catheter.

Preferably, the second balloon is substantially spherical when inflated.

In one aspect of the invention the display means is hard wired to the signal processor.

The invention also provides a method for monitoring the degree of opening of a ripening cervix of a female mammal, the method comprising monitoring a pressure indicative of the pressure of a compressible inflating medium in a first balloon inflated to a first predefined pressure located in the cervix, and determining the degree of opening of the cervix from the monitored pressure.

In one aspect of the invention the first balloon is inflated with the inflating medium to the first predefined pressure subsequent to being located in the cervix.

In another aspect of the invention the inflating medium is retained in the first balloon subsequent to inflating thereof in order to prevent deflating of the first balloon.

Preferably, the inflating medium is releasably retained in the first balloon. Advantageously, the inflating medium is retained in the first balloon by a first valve. Ideally, the first valve comprises a non-return valve.

Advantageously, the first valve comprises a releasable valve. Preferably, the first valve comprises a manually releasable valve.

In one aspect of the invention the first predefined pressure to which the first balloon is inflated is sufficient so that as the degree of opening of the cervix increases to a predefined degree of opening, the first balloon remains in abutting contact with the cervix wall defining the opening extending through the cervix.

In another aspect of the invention the first predefined pressure to which the first balloon is inflated is sufficient so that the pressure of the inflating medium in the first balloon remains positive relative to the external pressure acting on the first balloon by the cervix during the period the cervix is opening to the predefined degree of opening.

In another aspect of the invention the predefined degree of opening of the cervix corresponds to the degree of opening of the cervix in the fully ripened state indicative of the onset of labour.

Preferably, the first predefined pressure to which the first balloon is inflated is insufficient to effect opening of the cervix during ripening thereof.

In another aspect of the invention the pressure indicative of the pressure of the inflating medium in the first balloon is monitored by a pressure sensing means.

Preferably, the pressure sensing means is configured to produce a signal indicative of the pressure of the inflating medium within the first balloon.

In one aspect of the invention the pressure sensing means is located within the first balloon.

In an alternative aspect of the invention the pressure sensing means is located externally of the first balloon, and is configured to communicate with a conduit communicating with the first balloon.

In a further aspect of the invention the degree of opening of the cervix is determined from a correlating means which correlates a plurality of reference pressure values of an inflating medium in a reference balloon located in a reference cervix with corresponding values of reference degrees of opening of the reference cervix.

In one aspect of the invention a ripened alert signal is produced in response to the cervix reaching a fully ripened state.

In another aspect of the invention the ripened alert signal is presented in the form of a human sensory perceptible signal.

In one aspect of the invention an approaching ripening signal is produced in response to the cervix approaching the fully ripened state.

Preferably, the approaching ripening signal is presented in the form of a human sensory perceptible signal.

Preferably, a signal indicative of the degree of opening of the cervix is produced at predefined time intervals.

In another aspect of the invention the signals indicative of the degree of opening of the cervix are produced in the form of human sensory perceptible signals.

In another aspect of the invention each human sensory perceptible signal comprises one or more of a visually perceptible signal, an aurally perceptible signal, a tactilely perceptible signal.

Preferably, the tactilely perceptible signal comprises inducing vibration in a part of the body of the female mammal.

Advantageously, the vibration is induced in the vagina of the female mammal.

In another aspect of the invention the human sensory perceptible signal indicative of the degree of opening of the cervix comprises a visually perceptible signal.

Preferably, the visually perceptible signal indicative of the degree of opening of the cervix comprises one of a representation of the degree of opening of the cervix and indicia indicative of the degree of opening of the cervix.

In another aspect of the invention the representation of the cervix comprises a graphical representation of the opening through the cervix from the uterus to the vagina superimposed on a graphical representation of the opening of a fully ripened reference cervix from the uterus to the vagina.

Advantageously, the visual perceptible signal indicative of the degrees of opening of the cervix comprises a visual scale indicative of the degrees of opening of a reference cervix during ripening of a reference cervix from commencement of ripening to full ripening thereof, and a visual indication on the scale of the current degree of opening of the cervix.

Preferably, the visually perceptible representation of the degree of opening of the cervix comprises displaying a representation of the opening through the cervix on a colour chart.

Advantageously, the colours or the shades of colour of the colour chart progressively change outwardly from opposite sides of a centre line and a representation of the opening through the cervix is superimposed on the colour chart with the centre line of the opening coinciding with the centre line of the colour chart, and the representation is refreshed at the predefined time intervals, so that as the cervix ripens the boundary lines defining the opening through the cervix of the representation thereof progressively move outwardly from the centre line and through the progressively changing colours or shades thereof.

Preferably, the colours or shades of colour progressively transition from lighter colours or lighter shades to darker colours or darker shades from the centre line outwards.

In another aspect of the invention the signal indicative of the degree of opening of the cervix is communicated to a display means.

Preferably, the signal indicative of the degree of opening of the cervix is communicated wirelessly to a display means.

Advantageously, the signal indicative of the degree of opening of the cervix is communicated in one of a Bluetooth protocol and an NFC protocol.

Preferably, the signal indicative of the degree of opening of the cervix is configured for reception by a smart mobile device.

Advantageously, the signal indicative of the degree of opening of the cervix is configured for reception by a smart mobile device, the smart mobile device being one of Bluetooth and NFC enabled.

Preferably, the signal indicative of the degree of opening of the cervix is configured for reception by a smart mobile device configured by an app.

Advantageously, the signal indicative of the degree of opening of the cervix is configured for reception by a smart mobile phone configured by an app.

In one aspect of the invention the smart mobile device is configured by an app for receiving the signal indicative of the degree of opening of the cervix, and the smart mobile device is configured by an app to display one of the representation indicative of the degree of opening of the cervix and indicia indicative of the degree of opening of the cervix.

Preferably, the smart mobile device is configured by an app to receive and interpret the signal indicative of the degree of opening of the cervix, and to display the one of the representation indicative of the degree of opening of the cervix and indicia indicative of the degree of opening of the cervix.

Advantageously, the first balloon is located in the cervix extending the length of the cervix.

Preferably, a distal portion of the first balloon is configured to extend into the uterus of the female mammal, and to engage the uterus adjacent the cervix.

Advantageously, a proximal portion of the first balloon is configured to extend into the vagina, and to engage a portion of the cervix adjacent the vagina.

In a further aspect of the invention a second balloon is located on the catheter distally of the first balloon and adjacent the first balloon, the second balloon being configured for locating in the uterus and for engaging the cervix adjacent the uterus to simulate the pressure of the head of a foetus within the uterus bearing on the cervix.

Preferably, the first balloon comprises a proximal portion thereof configured to extend into the vagina and being engageable with a portion of the cervix adjacent the vagina, the proximal portion of the first balloon co-operating with the second balloon, so that the engagement action of the proximal portion of the first balloon with the portion of the cervix adjacent the vagina urges the second balloon into engagement with the cervix adjacent the uterus to simulate the pressure of the head of a foetus within the uterus bearing on the cervix.

Advantageously, a force is applied to the second balloon in a direction outwardly relative to the vagina for urging the second balloon into engagement with the cervix adjacent the uterus.

In one aspect of the invention an urging means is provided for applying the outward force to the second balloon.

Preferably, the urging means is anchored to a part of the body of the female mammal.

Advantageously, the urging means is anchored externally of the vagina.

Preferably, the urging means is anchored adjacent the entrance to the vagina externally of the vagina.

In another aspect of the invention the urging means is anchored to one of the crotch and one of the legs of the female mammal.

Preferably, the urging means is secured to one of the catheter, the second balloon, and the first balloon.

In another aspect of the invention the second balloon is inflated with an inflating medium.

In a further aspect of the invention the inflating medium with which the second balloon is inflated is the same or different to the inflating medium with which the first balloon is inflated.

In a further aspect of the invention the inflating medium with which the second balloon is inflated is one of a compressible fluid and an incompressible fluid.

In one aspect of the invention the second balloon is substantially spherical when inflated.

In another aspect of the invention the portion of the first balloon which is configured for locating in the opening extending through the cervix between the uterus and the vagina is of substantially cylindrical shape when inflated.

Further the invention provides apparatus configured to carry out the method according to the invention for monitoring the degree of opening of the cervix in a female mammal.

The advantages of the invention are many. One particularly important advantage of the invention is that by using the method and apparatus and the balloon catheter according to the invention, in the case of human female, there is no need for the subject to be hospitalised during monitoring of the ripening of the cervix. The subject, the ripening of the cervix of which is being monitored, can proceed about her normal business, since the apparatus is self-contained, and those parts of the apparatus which are not located in the uterus, cervix and vagina of the subject can be worn by the subject. Indeed, where the apparatus is provided with a balloon catheter whereby the housing which houses the signal processing means and the communicating means is located on the catheter and configured to be located within the vagina, and the proximal end of the catheter terminates just externally of the vagina of the subject, the entire apparatus, including the balloon catheter is located entirely internally within the subject.

A further advantage of the apparatus and the balloon catheter as well as the method according to the invention is achieved where the balloon catheter comprises a second balloon, or alternatively, the first balloon comprises a distal portion configured to be located within the uterus and configured to bear on the cervix to simulate the head of a foetus. The advantage of this aspect of the apparatus, balloon catheter and method is that as well as monitoring the ripening of the cervix, the action of the second balloon or the distal portion of the first balloon assists in the ripening of the cervix.

A further advantage of providing a second balloon on the balloon catheter for locating in the uterus, or providing the first balloon with a distal portion for locating in the uterus whereby either the second balloon or the distal portion of the first balloon are configured to bear on the cervix and to simulate the action of the head of a foetus is that in pregnancies where the head of the foetus is not properly engaged with the cervix, the action of the second balloon on the cervix or the distal end of the first balloon on the cervix simulating the head of the foetus further assists ripening of the cervix.

The invention will be more clearly understood from the following description of some preferred embodiments thereof, which are given by way of example only, with reference to the accompanying drawings, in which:
Fig. 1 illustrates a transverse cross-sectional side elevational view of a pelvic portion of a human female subject illustrating apparatus and a balloon catheter, both according to the invention, in use for monitoring the degree of opening of the cervix of the human female subject during ripening of the cervix prior to the onset of labour,
Fig. 2 is a transverse cross-sectional side elevational view of the balloon catheter of Fig. 1,
Fig. 3 illustrates a look-up table of reference pressure values and reference volume values obtained during simulated ripening of a simulated reference cervix,
Fig. 4 illustrates a display of a representation of an opening extending through a cervix illustrating the degree of opening of the cervix during ripening thereof,
Fig. 5 illustrates a display of a graphical representation of the degree of opening of a cervix during ripening thereof,
Fig. 6 illustrates a portion of the simulated reference cervix,
Fig. 7 illustrates a table showing how the reference pressure values and reference volume values of the look-up table of Fig. 3 have been derived,
Fig. 8 illustrates a plot of reference pressure values against corresponding reference volume values obtained during the simulated ripening of the simulated reference cervix,
Fig. 9 is a view similar to Fig. 1 illustrating a balloon catheter according to another embodiment of the invention, for monitoring the degree of opening of the cervix of a human female subject during ripening of the cervix prior to the onset of labour,
Fig. 10 is a cross-sectional side elevational view similar to Fig. 2 of the balloon catheter of Fig. 9,
Fig. 11 is a view similar to Fig. 1 illustrating a balloon catheter according to another embodiment of the invention, for monitoring the degree of opening of the cervix of a human female subject during ripening of the cervix prior to the onset of labour,
Fig. 12 is a cross-sectional side elevational view similar to Fig. 2 of the balloon catheter of Fig. 11,
Fig. 13 is a view similar to Fig. 1 illustrating apparatus and a balloon catheter, both according to another embodiment of the invention for monitoring the degree of opening of the cervix of a human female subject during ripening of the cervix prior to the onset of labour,
Fig. 14 is a block representation of circuitry of the apparatus of Fig. 13,
Fig. 15 is a cross-sectional side elevational view of a device not according to the invention for stimulating ripening of the cervix of a female human subject, and
Fig. 16 is a view similar to Fig. 1 of apparatus according to another embodiment of the invention for use in monitoring the degree of opening of the cervix of a human female subject during ripening of the cervix prior to the onset of labour.

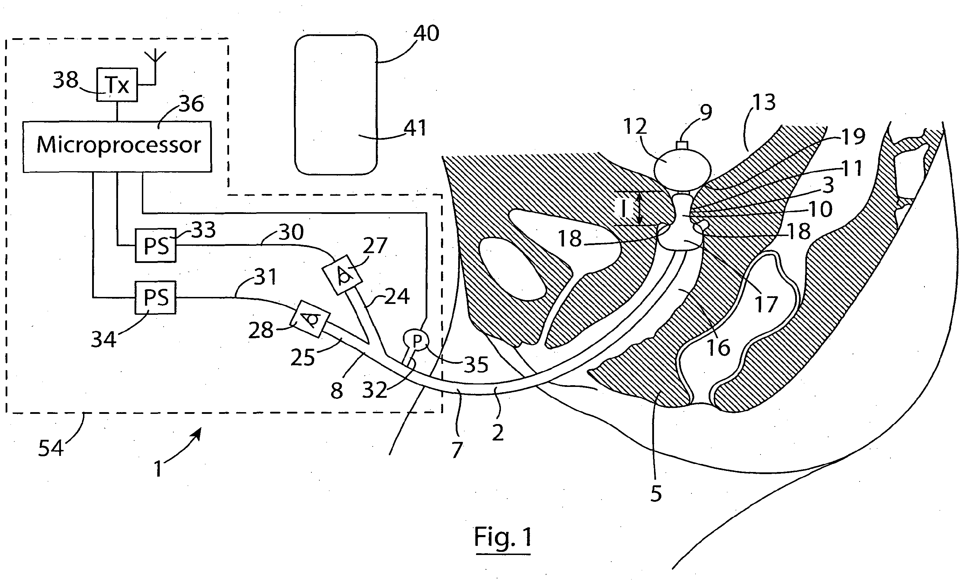

Referring to the drawings, and initially to Figs. 1 to 5 thereof, there is illustrated apparatus according to the invention, indicated generally by the reference numeral 1, for monitoring the degree of opening of the cervix 3 in a female mammal, in this case a female human subject as the cervix 3 ripens prior to the onset of labour in a pregnancy. The apparatus 1 comprises a balloon catheter also according to the invention, indicated generally by the reference numeral 2, configured for monitoring the degree of opening of the cervix 3 of the female subject, the pelvic area only of the subject is illustrated in Fig. 1, and is indicated by the reference numeral 5.

The balloon catheter 2 comprises an elongated catheter 7 extending between a proximal end 8 and a distal end 9. A first balloon, namely, an elongated cervix engaging balloon 10 of substantially cylindrical shape when inflated is located on the catheter 7 towards the distal end 9 of the catheter 7 for locating in the opening 11 extending through the cervix 3 for monitoring the degree of opening of the cervix 3, as will be described in detail below. A second balloon, namely, a distal balloon 12 of substantially spherical shape when inflated is located on the catheter 7 adjacent the distal end 9 thereof, and is located distally of the cervix engaging balloon 10, but adjacent thereto. The distal balloon 12 is configured for locating in the uterus 13 of the subject adjacent the cervix 3 for simulating the head of a foetus (not shown) in the uterus 13 bearing on the cervix 3, as will be described below. The catheter 7 is of any suitable material, and the cervix engaging balloon 10 and distal balloon 12 are of a flexible, elastic, and a relatively easily expandable material, in this embodiment of the invention the cervix engaging balloon 10 is of polyurethane, and the distal balloon 12 is of silicone.

The catheter 7 extends through the cervix engaging balloon 10 and the distal balloon 12 and defines with the balloons 10 and 12 respective annular hollow interior regions 14 and 15, respectively, for accommodating an inflating medium, which in this embodiment of the invention comprises a compressible fluid, namely, air. Although it will be readily appreciated by those skilled in the art that the inflating medium may be any suitable gas, and in the case of the distal balloon 12 the inflating medium may be an incompressible fluid, for example, a saline solution.

The cervix engaging balloon 10 is of sufficient length *L* to extend through the length *l* of the opening 11 extending through the cervix 3 from the uterus 13 to the vagina 16 prior to the commencement of ripening of the cervix 3, which is when the opening 11 thereof is at its longest length *l.* The length *L* of the cervix engaging balloon 10 is also sufficient, so that a proximal portion 17 of the cervix engaging balloon 10 extends from the cervix 3 into the vagina 16. Thus when the cervix engaging balloon 10 is inflated in the cervix 3, the proximal portion 17 of the cervix engaging balloon 10 is located in the vagina 16 and bears on a portion 18 of the cervix 3 adjacent the vagina 16. The action of the proximal portion 17 of the cervix engaging balloon 10 bearing on the portion 18 of the cervix 3 which extends into the vagina 16 tends to urge the distal balloon 12 when inflated into engagement with a portion 19 of the cervix 3 adjacent the uterus 13, in order to simulate the pressure of the head of a foetus (not shown) in the uterus 13 bearing on the cervix 3, and to stimulate ripening of the cervix.

In this embodiment of the invention the cervix engaging balloon 10 is of axial length *L* of 80mm, and can inflate to a diameter of approximately 50mm, when inflated to a pressure of approximately 80mmHg. Since the normal length *l* of the opening 11 of the cervix 3 is typically 40mm to 50mm prior to the commencement of ripening thereof, the distal 40mm to 50mm of the cervix engaging balloon 10 is located in the opening 11 of the unripened cervix, and the remaining 30mm to 40mm which forms the proximal portion 17 of the cervix engaging balloon 10 is located in the vagina. Typically, the normal internal diameter of the opening 11 extending through the cervix 3 of a typical cervix of a typical female human subject prior to the commencement of ripening is between 5mm and 10mm. When the cervix has opened or ripened fully due to the onset of labour, the internal diameter of the opening 11 extending through the cervix 3 increases until the minimum internal diameter of the opening 11 extending through the cervix 3 is approximately 40mm, and the volume of the opening 11 of a fully ripened cervix 3 would be in the order of approximately 63cms³.

The distal balloon 12 is typically inflated to a diameter of the order of 40mm to 50mm.

A first lumen 20 for accommodating inflating medium to the cervix engaging balloon 10 extends longitudinally through the catheter 7 from the proximal end 8 thereof and terminates in the catheter 7 within the cervix engaging balloon 10. A plurality of first communicating ports 21 extending radially through the catheter 7 from the first lumen 20 communicate with the hollow interior region 14 of the cervix engaging balloon 10 for accommodating the inflating medium from the first lumen 20 into the hollow interior region 14 of the cervix engaging balloon 10.

A second lumen 22 for accommodating inflating medium to the distal balloon 12 extends longitudinally through the catheter 7 from the proximal end 8 and terminates in the catheter 7 within the distal balloon 12. A plurality of second communicating ports 23 extend radially from the second lumen 22 through the catheter 7 and communicate with the hollow interior region 15 of the distal balloon 12 for accommodating inflating medium from the second lumen 22 into the hollow interior region 15 of the distal balloon 12.

The proximal end 8 of the catheter 7 is branched to form a first branch 24 through which the first lumen 20 extends, and a second branch 25 through which the second lumen 22 extends. The first and second branches 24 and 25 terminate in first and second retaining means, in this case releasable retaining means for releaseably retaining the inflating medium in the respective balloons 10 and 12; in this embodiment of the invention the first and second retaining means comprise manually releasable first and second non-return valves 27 and 28, respectively. The first and second lumens 20 and 22 are coupled through the first and second non-return valves 27 and 28 by first and second conduits 30 and 31, respectively, to first and second pressure sources for providing the inflating medium for the balloons 10 and 12; in this embodiment of the invention, the first and second pressure sources comprise first and second pumps 33 and 34, respectively. The first pump 33 is configured for inflating the cervix engaging balloon 10 with the compressible inflating medium, namely, air. The second pump 34 is configured for inflating the distal balloon 12 with either a compressible or an incompressible inflating medium, in this case also air, although the distal balloon 12 may also be inflated with an incompressible fluid, such as a saline solution. The first and second non-return valves 27 and 28 are configured to facilitate inflating of the cervix engaging balloon 10 and the distal balloon 12, respectively, and to seal and retain the inflating medium in the respective balloons 10 and 12, in order to prevent deflating of the balloons 10 and 12, unless either or both of the first and second non-return valves are manually operated to release the inflating medium from the corresponding one of the cervix engaging balloon 10 and the distal balloon 12.

A pressure sensing means comprising a pressure sensor 35, typically, a piezo-electric pressure sensor is tapped into the first lumen 20 in the catheter 7 adjacent the proximal end 8 thereof by a conduit 32 for monitoring the pressure of the inflating medium in the first lumen 20, which is indicative of the pressure of the inflating medium in the cervix engaging balloon 10. The pressure sensor 35 produces an electrical signal which is indicative of the pressure of the inflating medium in the cervix engaging balloon 10.

A signal processing means, in this embodiment of the invention a signal processor, which is provided by a microprocessor 36 is programmed to control the operation of the apparatus 1. The microprocessor 36 is programmed to operate the first pump 33 and the second pump 34 for inflating the cervix engaging balloon 10 and the distal balloon 12, and to read the signal from the pressure sensor 35 in order to determine the pressure of the inflating medium in the cervix engaging balloon 10.

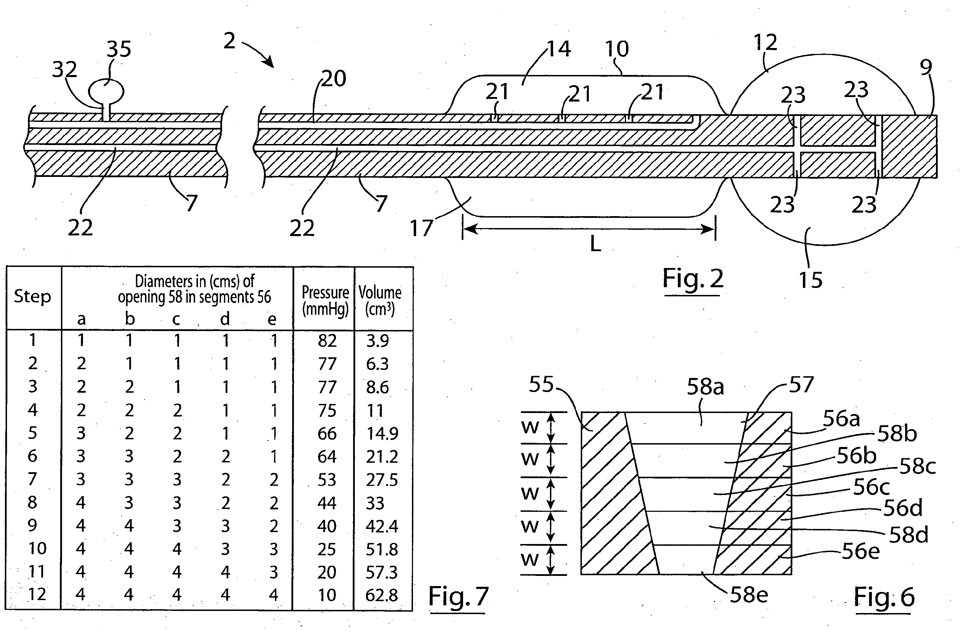

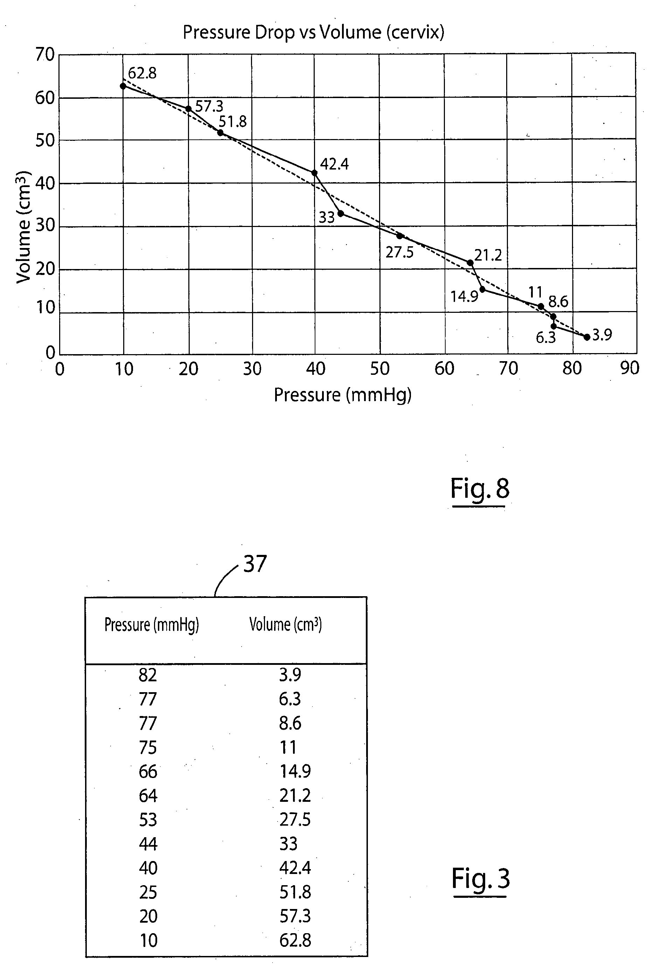

A correlating means for correlating the signals indicative of the pressure of the inflating medium in the cervix engaging balloon 10 with corresponding values of the degree of opening of the cervix, which in this embodiment of the invention are corresponding empirically derived values of the volume of the opening 11 extending through a reference cervix along the length 1 of the reference cervix as the reference cervix progressively ripens from a state prior to the commencement of ripening to the fully ripened state, in this embodiment of the invention comprises a look-up table 37, see Fig. 3. The reference cervix from which the pressure and volume values in the look-up table 37 have been derived, in this embodiment of the invention, is a simulated reference cervix in which the opening through the simulated cervix was increased in a manner similar to the manner in which the opening extending through a typical cervix would increase during ripening thereof. Such a reference cervix and the derivation of the look-up table 37 is described below with reference to Figs. 6 and 7. The pressure values of the inflating medium in a balloon similar to the cervix engaging balloon located in the simulated reference cervix were recorded as the opening extending through the simulated reference cervix was increased in incremental steps as will be described below. Thus, in the look-up table 37 illustrated in Fig. 3 the volume values of the opening extending through the simulated reference cervix as the volume of the opening was incrementally increased are set forth in column 2 of the look-up table 37, and the corresponding pressure values of the inflating medium in the balloon which was located in the opening extending through the simulated reference cervix are set forth in column 1 of the look-up table 37, and cross-referenced with the corresponding volume values in column 2 of the look-up table 37. As will be described below, in deriving the pressure values set forth in column 1 of the look-up table 37, the balloon was initially inflated to a first predefined pressure of approximately 80mm Hg and sealed when the simulated reference cervix was at its minimum diameter corresponding to a typical cervix prior to the commencement of ripening. As the volume of the opening through the simulated reference cervix was incrementally increased, the pressure of the inflating medium in the balloon fell in corresponding increments, since the balloon was sealed.

The microprocessor 36 is programmed to read the signal from the pressure sensor 35 which is indicative of the pressure of the inflating medium in the cervix engaging balloon 10 at predefined time intervals typically in the range of 1 second to 5 minutes duration, and more typically, of 60 seconds duration. The microprocessor 36 is programmed so that on reading the signal from the pressure sensor 35 at the end of each predefined time interval, the microprocessor 36 reads the volume value from the look-up table 37, which corresponds most closely with the pressure read from the pressure sensor 35. The microprocessor 36 then produces a signal indicative of the degree of opening of the cervix 3, which in this embodiment of the invention comprises a digital data package which comprises data indicative of the current volume of the opening 11 extending through the cervix 3 along the length 1 thereof and the current value of the pressure of the inflating medium in the cervix engaging balloon 10.

A communicating means, in this case a wireless communications module 38 is operated under the control of the microcontroller 36, and is configured to communicate in Bluetooth protocol, a near field communications protocol or other suitable wireless protocol with a suitably enabled smart mobile device. In this case the communications module is configured to communicate in a Bluetooth protocol, and the smart mobile device comprises a Bluetooth enabled smart mobile phone 40. The smart mobile phone 40 is paired with the communications module 38, and is configured by a suitable app for communicating with the communications module 38. The microprocessor 36 is programmed so that on determining the current volume of the opening 11 extending through the cervix 3 along the length thereof from the look-up table 37 at the end of each predefined time interval, the microprocessor 36 operates the communications module 38 to transmit the digital data package indicative of the current volume of the opening 11 extending through the cervix 3 along the length thereof and the current value of the pressure of the inflating medium in the cervix engaging balloon 10, for reception by the mobile phone 40. The mobile phone 40 is configured by the app to display one of a representation indicative of the degree of opening of the cervix 3 and indicia indicative of the degree of opening of the cervix 3 on a visual display screen 41 of the mobile phone 40, and to refresh the representation or the indicia in response to each digital data package received from the communications module 38 indicative of the current volume of the opening through the cervix 3.

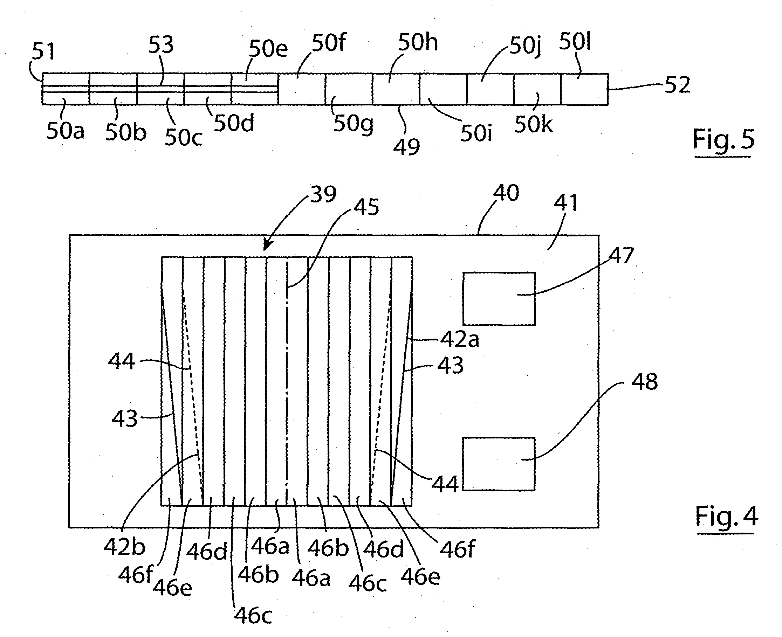

Referring now to Fig. 4, an illustration of a display 39 of representation 42 of two states of the opening 11 through of the cervix 3 is illustrated on the visual display screen 41 of the smart mobile phone 40. One of the representations 42a is illustrated in full lines 43 and the other one of the representations 42b is illustrated in broken lines 44. The display 39 is divided by a centre line 45 which extends down through the display 39. A colour chart comprising bands 46 of either different colours or different shades of the one colour extend parallel to the centre line 45 on respective opposite sides of the centre line 45. Typically, when the bands 46 are of different shades of the one colour, the darkness of the shades of the bands 46 progressively increases in a direction outwardly from the centre line 45 on each side thereof. In this case the bands 46a which are adjacent the centre line 45 are of the lightest shade of the colour, while the bands 46f which are furthest away from the centre line 45 are of the darkest shade of the colour. The representations 42 of the state of the opening 11 through the cervix 3 is displayed on the screen 41 with the centre line of the representation of the opening 11 extending through the cervix 3 coinciding with the centre line 45 of the display 39. When the opening 11 through the cervix 3 is in a state prior to the commencement of ripening, the representation 42 of the cervix 3 would be displayed within the bands 46a. As the cervix 3 progressively opens during ripening thereof, the full lines 43 of the representation 42 representing the opening 11 extending through the cervix 3 progressively move outwardly from the centre line 45 through the bands 46 of the progressively darkening shades of the colour, in other words from the bands 46a to the bands 46f, thus indicating the progressive opening of the opening 11 through the cervix 3. The bands 46f are of the darkest shade of the colour, and when the pressure read from the pressure sensor 35 by the microprocessor 36 is indicative of the cervix 3 being fully ripened, the digital data package transmitted by the communications module 38 to the mobile phone 40 results in the full lines 43 of the representation 42 representing the opening 11 extending through the cervix 3 being displayed in the bands 46f of the darkest shade of the colour, as illustrated in Fig. 4, thus indicating that the volume of the opening 11 through the cervix 3 is of maximum volume and is indicative of the cervix 3 being in the fully ripened state just prior to the onset of labour. The representation 42 of the cervix 3 illustrated in the display 39 in the broken lines 44 illustrates the state of the opening 11 extending through the cervix 3 as the cervix 3 is approaching the fully ripened state.

If the display 39 were provided as a multi-colour colour chart with the bands 46 being of different colours, in general, the bands 46a closest to the centre line 45 would be of a relatively light colour and the colours of the bands 46b to 46f would be of progressively darkening colours.

The value of the current volume of the opening 11 extending through the cervix 3 is displayed in a window 47 in the screen 41, and the value of the current pressure of the inflating medium in the cervix engaging balloon 10 is displayed in the window 48.

Referring now to Fig. 5, an alternative method for representing the degree of opening of the cervix 3 on the screen 41 of the smart mobile phone 40 is illustrated. In this representation, the smart mobile phone 40 displays a scale 49 in the form of an elongated band which may be a single colour scale with the shade of the colour progressively increasing from a light shade to a dark shade in increments 50a to 501 from a zero end 51 of the scale 49 to the end 52, which represents a fully ripened cervix 3. Alternatively, the scale may be a multi-colour scale, with the colours of the scale progressively darkening in increments, similar to the increments 50. A line 53 is progressively drawn by the app, with which the smart mobile phone 40 is configured, along the scale 49 in response to the respective digital data package received by the smart mobile phone 40 from the communications module 38 to indicate the current degree of opening of the cervix 3 as the opening 11 extending through the cervix 3 progressively opens from the state prior to the commencement of ripening thereof which corresponds to the zero end 51 of the scale 49 to the end 52 of the scale 49 which represents the cervix 3 being fully ripened.

In this embodiment of the invention the pressure sensor 35, the microprocessor 36 and the communications module 37, as well as the first and second pumps 33 and 34 are located in a housing 54 which is illustrated in broken lines in Fig.1. The housing 54 is of size so that the housing 54 may be worn by the subject, or placed in a pocket of a garment of the subject.

In use, the balloon catheter 2 is urged through the vagina 16 of the subject with the cervix engaging balloon 10 and the distal balloon 12 deflated until the distal balloon 12 is just inside the uterus 13. Under the control of the microprocessor 36, the second pump 34 is operated to inflate the distal balloon 12 with air. In this embodiment of the invention the distal balloon 12 is inflated to a diameter of between 40mm and 50mm. With the distal balloon 12 inflated, the catheter is lightly pulled in a generally outwardly direction relative to the vagina 16 in order to locate the distal balloon 12 in the uterus 13 abutting the cervix 3. By locating the distal balloon 12 in the uterus 13 abutting the cervix 3, the cervix engaging balloon 10 is correctly located in the cervix 3. The first pump 33 is operated under the control of the microprocessor 36 to inflate the cervix engaging balloon 10 with air to a pressure of approximately 80mmHg.

On completion of inflating the cervix engaging balloon 10 and the distal balloon 12, the first and second pumps 33 and 34 are deactivated by the microprocessor 36, and the inflating medium in the cervix engaging balloon 10 and the distal balloon 12 is retained therein by the first and second non-return valves 27 and 28.

The microprocessor 36 reads the signal from the pressure sensor 35 at the end of each predefined time interval. The microprocessor 36 then determines the current volume of the opening 11 extending through the cervix 3 along the length 1 thereof from the look-up table 37 by reading the volume value from column 2 of the look-up table 37 which corresponds most closely with the pressure indicative of the pressure of the inflating medium in the cervix engaging balloon 10 read from the pressure sensor 35. The digital data package containing data indicative of the current volume of the opening 11 extending through the cervix 3 and the current pressure of the inflating medium in the cervix engaging balloon 10 is prepared by the microprocessor 36 and transmitted by the communications module 38 under the control of the microprocessor 36 at the end of each predefined time interval in Bluetooth protocol for reception by the paired Bluetooth enabled smart mobile phone 40.

The smart mobile phone 40 under the control of the app, on receiving the first digital data package, displays the representation 42 of the opening 11 of the cervix 3 on the display screen 41 superimposed on the colour chart of the display 39 illustrated in Fig. 4. The representation 42 of the opening 11 extending through the cervix 3 displayed on the screen 41 of the mobile phone 40 is refreshed in response to each digital data package received from the communications module 38 at the end of each predefined time interval.

The numerical value of the volume of the opening 11 extending through the cervix 3 is displayed in the window 47 on the display screen 41 of the smart mobile phone 40 under the control of the app, and is refreshed at the end of each predefined time interval. The numerical value of the pressure of the inflating medium in the cervix engaging balloon 10 is displayed in the window 48 on the display screen 41 of the smart mobile phone 40, and refreshed at the end of each predefined time interval.

By periodically monitoring the smart mobile phone, the subject knows the degree to which her cervix 3 has opened, and thus, can identify the time to commence her journey to the maternity hospital so that she will arrive in time to give birth to the baby in the maternity hospital.

Additionally, in this embodiment of the invention the microprocessor 36 is programmed to produce two alert signals, namely, a ripened alert signal indicative of the cervix 3 being fully ripened in response to the pressure read from the pressure sensor 35 being indicative of the cervix being fully ripened, and an approaching ripening alert signal in response to the cervix approaching the fully ripened state. Typically, the approaching ripened alert signal is produced by the microprocessor 36 on the microprocessor 36 determining the volume of the opening 11 extending through the cervix 3 is at a volume of the order of 75% to 80% of the maximum volume corresponding to the cervix being fully ripened. The microprocessor 36 operates the communications module 38 to transmit the approaching ripening alert signal and the ripened alert signal. The app in the smart mobile phone 40 configures the smart mobile phone 40 to allow two ringtones to be selected, one of the selected ringtones to be activated in response to receipt of the approaching ripening alert signal, and the other one of the ringtones to be activated in response to receipt of the ripened alert signal.

By providing the cervix engaging balloon 10 to be of a flexible elastic polyurethane material which is relatively easily expandable and to be of length L of approximately 80mm, and by inflating the cervix engaging balloon 10 to the pressure of approximately 80mmHg, the proximal portion 17 of the cervix engaging balloon 10 expands to substantially fill the portion of the vagina 13 adjacent the cervix 3 and bears on the cervix 3 for urging the distal balloon 12 into engagement with the cervix 3 and retaining the distal balloon 12 in engagement with the cervix 3 to simulate the head of a foetus in the uterus 14 bearing on the cervix 3. Due to the volume and the elasticity of the material of the cervix engaging balloon 10 by inflating the cervix engaging balloon 10 to the pressure of only approximately 80mmHg, the balloon has no dilating effect on the cervix 3.

It has also been found that by inflating the cervix engaging balloon 10 with air to the pressure of 80mmHg when the cervix 3 is in the unripened or unopened state, in other words is in a state prior to the commencement of ripening, as the cervix 3 ripens and the cervix engaging balloon 10 expands to fill the opening 11 through the ripening cervix 3, the pressure of the air in the cervix engaging balloon 10 falls. However, the pressure of the air in the cervix engaging balloon 10 remains positive. Thus, as the degree of opening of the cervix 3 increases, and the portion of the cervix engaging balloon 10 in the opening 11 extending through the cervix 3 expands with the cervix 3, the portion of the cervix engaging balloon 10 in the opening 11 of the cervix 3 remains in contact with the cervix 3. In this way the pressure of the air in the cervix engaging balloon 10 is inversely proportional to the degree of opening of the cervix 3 until the degree of opening of the cervix 3 corresponds to a fully ripened cervix. Therefore, by monitoring the pressure of the compressible inflating medium in the cervix engaging balloon 10, the degree of opening of the cervix 3 can be readily determined.

It is envisaged that in certain cases, the first and second pumps 33 and 34 may be dispensed with, and the first and second non-return valves 37 and 38 could, for example, terminate in respective luer couplers for releasably coupling to respective medical syringes (not shown) for manually inflating the cervix engaging balloon 10 and the distal balloon 12. The syringes would be disconnected from the first and second non-return valves 27 and 28 on completion of inflating of the distal balloon 12 and the cervix engaging balloon 10. In cases where the pumps 33 and 34 are dispensed with, the housing 53 would only house the pressure sensor 35, the microprocessor 36 and the communications module 38.

It is also envisaged that instead of or as well as the communications module 38, a visual display screen may be provided on the housing 54, which could be hardwired to the microprocessor 36, and which would display a representation of the opening through the cervix and the volume of the opening extending through the cervix and the pressure of the inflating medium in the cervix engaging balloon 10, or other suitable representation of the opening through the cervix as already described with reference to Figs. 4 and 5 for display on the smart mobile phone. Additionally, a buzzer or other suitable sounder for producing audible signals under the control of the microprocessor 36 in response to the ripened alert signal and the approaching ripening alert signal may be located in the housing 54.

Turning now to the derivation of the look-up table 37, and referring to Figs. 6 to 8, the values set forth in the look-up table 37 of Fig. 3, as discussed above, have been derived from a simulation of a ripening reference cervix. The volume of the simulated reference cervix was varied over its length 1 from 3.9cm³ to 62.8cm³ in order to simulate a typical ripening cervix. The reference cervix 55 is illustrated in Fig. 6 and was formed by five segments 56 along its length of similar width w. An opening 57 extending through the reference cervix 55 is formed by openings 58 formed in the segments 56, and represents the opening extending through a cervix 3. The varying of the volume of the opening 57 extending through the reference cervix 55 was achieved by progressively increasing the diameter of the openings 57 formed in the segments 56 from the segment 56a at the end of the reference cervix corresponding to the end of a cervix adjacent the uterus to the segment 56e at the end of the reference cervix corresponding to the end of a cervix adjacent the vagina, in twelve steps, namely, steps 1 to 12, see Fig. 7.

In a normally ripening cervix, the length 1 of the cervix decreases as ripening progresses, and the opening through the cervix progressively opens from the top adjacent the uterus to the bottom adjacent the vagina. Accordingly, in order to simulate a ripening cervix the openings 58 formed in the segments 56 were opened in the sequence set out in Fig. 7 in columns 2 to 6 in the twelve steps set out in column 1 of Fig. 7.

In each step of the steps 1 to 12, the diameters of the openings 58 of the segments 56 were progressively increased from step 1 to step 12. The volume of the opening 57 through the simulated reference cervix 55 at step 1 corresponds to a typical cervix before the commencement of ripening. At step 12 the volume of the opening 57 through the simulated reference cervix 55 corresponds to the volume of a typical fully ripened cervix. At step 1 the opening 57 through the reference cervix 55 was essentially cylindrical from the opening 58a to the opening 58e at 1cm diameter, through step 2 where the diameter of the opening 58a was increased to 2cm, while the diameter of the openings 58b to 58e was maintained at 1cm. In step 3 the diameter of the openings 58a was retained at 2cm, but the diameter of the opening 58b was increased to 2cm, while the diameters of the openings 58c to 58e were maintained at 1cm, and so on until step 10 when the diameters of the openings 58a to 58c had been increased to 4cm and the diameters of the openings 58d to 58e were at 3cm. At step 11 the diameter of the opening 58d was increased to 4cm, while the diameters of the openings 58a to 58c were held at 4cm and the diameter of the opening 58e was held at 3cm. In step 12 the diameter of the opening 58e was increased to 4cm, while the diameters of the openings 58a to 58d were retained at 4cm, thus simulating a cylindrical opening 57 through the reference cervix 55 of diameter of 4cm, which is indicative of a fully ripened cervix.

A balloon similar to the cervix engaging balloon 10 was located in the simulated reference cervix 55 of Fig. 6 and inflated with air to a pressure of approximately 80mmHg. The pressure values of the air pressure in the balloon were recorded at each of steps 1 to 12 in mmHg and appear in column 7 of the table of Fig. 7, while the corresponding volume values of the opening 57 extending through the simulated reference cervix 55 through the twelve steps appear in column 8 of the table of Fig. 7.

Fig. 8 illustrates a graph of a plot of the pressure values from column 7 of the table of Fig. 7 of the inflating medium in the balloon located in the reference cervix 55 plotted against the volume of the opening 57 extending through the simulated reference cervix 55 from column 8 of the table of Fig. 7 as the degree of opening of the simulated reference cervix 55 increases. The pressure of the inflating medium in the balloon in mmHg is plotted on the X-axis, while the volume of the opening 57 extending through the simulated reference cervix 55 is plotted on the Y-axis in cm³. As can be seen, the relationship between the pressure of the inflating medium in the balloon and the volume of the simulated cervix is substantially linear.

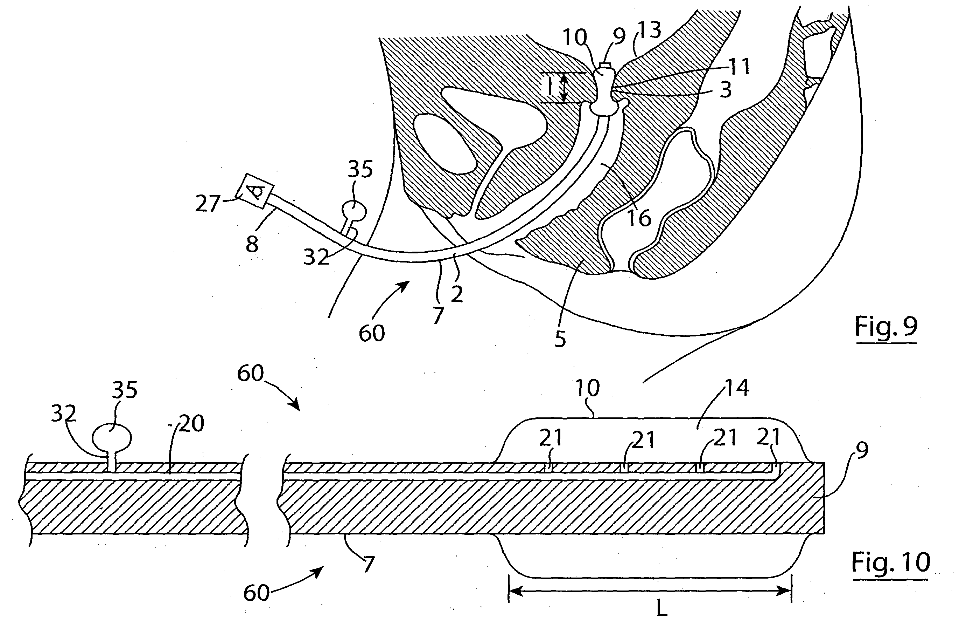

Referring now to Figs. 9 and 10, there is illustrated a balloon catheter according to another embodiment of the invention, indicated generally by the reference numeral 60, which is configured for monitoring the degree of opening of the cervix 3 of a female human subject during ripening of the cervix 3 prior to the onset of labour. The balloon catheter 60 is substantially similar to the balloon catheter 2 described with reference to Figs. 1 and 2, and similar components are identified by the same reference numerals. The balloon catheter 60 is also suitable for use with the apparatus 1, which is also according to the invention and which has been described with reference to Figs. 1 and 2. The main difference between the balloon catheter 60 and the balloon catheter 2 is that the distal balloon has been omitted and the cervix engaging balloon 10 is located adjacent the distal end 9 of the catheter 7. Only a single lumen, namely, the first lumen 20 extends through the catheter 7 from the proximal end 8 and terminates within the cervix engaging balloon 10 for accommodating an inflating medium therethrough for in turn inflating the cervix engaging balloon 10.

Accordingly, in this embodiment of the invention the balloon catheter 60 is suitable only for monitoring the degree of opening of the cervix 3. Additionally, since the balloon catheter 60 is provided without the distal balloon 12, there is no need for the cervix engaging balloon 10 to have a proximal portion which extends into the vagina 16. Accordingly, in this embodiment of the invention the cervix engaging balloon 10 is of substantially similar length *L* to the length *l* of the opening 11 extending through the cervix 3 before the commencement of ripening of the cervix 3, and thus, is of length *L* in the order of 40mm to 50mm.

Otherwise, the balloon catheter 60 and its use is similar to the balloon catheter 2 described with reference to Figs. 1 and 2.

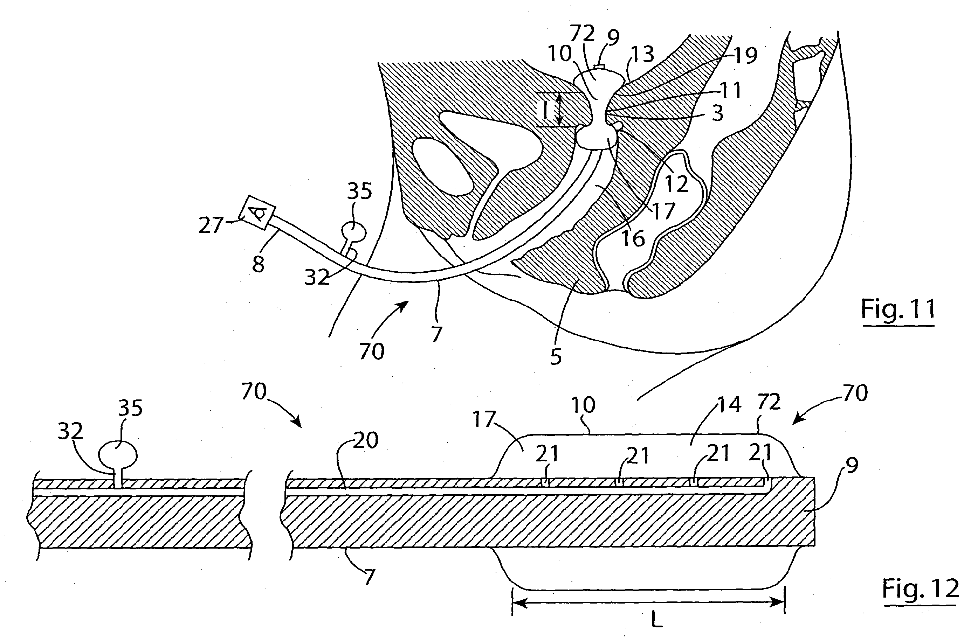

Referring now to Figs. 11 and 12, there is illustrated a balloon catheter according to another embodiment of the invention, indicated generally by the reference numeral 70, which is configured for monitoring the degree of opening of the cervix 3 of a female human subject during ripening of the cervix 3 prior to the onset of labour. The balloon catheter 70 is somewhat similar to the balloon catheter 2 described with reference to Figs. 1 and 2, and similar components are identified by the same reference numerals.

The main difference between the balloon catheter 70 and the balloon catheter 2 is that the distal balloon has been omitted, and the cervix engaging balloon 10 is located adjacent the distal end 9 of the catheter 7. Additionally, in this embodiment of the invention the cervix engaging balloon 10 is longer than the cervix engaging balloon 10 of the balloon catheter 2 of Figs. 1 and 2 and is of sufficient length *L* to extend through the cervix 3, and also to extend from the cervix 3 into both the uterus 13 and into the vagina 16. In this embodiment of the invention the length *L* of the cervix engaging balloon 10 is in the order of 120mm, and inflates to a diameter of approximately 40mm, and is constructed of a flexible elastic polyurethane material similar to that of the cervix engaging balloon 10 of the balloon catheter 2 of Figs. 1 and 2.

In this embodiment of the invention the balloon catheter 70 is inserted through the vagina 16 until the cervix engaging balloon 10 is located in the cervix 3'with approximately 40mm of the length *L* of the balloon 10, which forms a distal portion 72 of the cervix engaging balloon 10, located in the uterus 15, and approximately 3cm of the length *L* of the cervix engaging balloon 10 which forms the proximal portion 17 of the cervix engaging balloon 10 located in the vagina 16. The cervix engaging balloon 10 is located in the opening 11 extending through the cervix 3 when the cervix 3 is in the normal state prior to the commencement of ripening, and is inflated with air to a pressure of approximately 80mmHg. The pressure of approximately 80mmHg of the inflating medium in the cervix engaging balloon 10 is insufficient to have any dilating effect on the cervix 3. However, the pressure in the cervix engaging balloon 10 is sufficient to cause the distal portion 72 of the cervix engaging balloon 10, which is located in the uterus 13, and the proximal portion 17 of the cervix engaging balloon 10, which is located in the vagina 16, to co-operate with each other and with the cervix 3 to urge the distal portion 72 of the cervix engaging balloon 10 into engagement with the cervix 3 adjacent the uterus 13 in order to simulate the normal pressure of the head of a foetus in the uterus 13 bearing on the cervix 3 during ripening of the cervix 3. Accordingly, in this embodiment of the invention the balloon catheter 70 both acts to initiate the biological processes which leads to the opening of the cervix 3, and also allows monitoring of the degree of opening of the cervix 3 as the cervix 3 opens during ripening of the cervix 3 prior to the onset of labour.

The balloon catheter 70 is suitable for use with the apparatus 1 which has been described with reference to Figs. 1 and 2 with the exception that the second pump 34 is not required, and the first pump 33 is operated under the control of the microprocessor 36 to inflate the cervix engaging balloon 10 with air to a pressure of 80mmHg. Alternatively, the cervix engaging balloon 10 may be inflated by a syringe connected to the first non-return valve 27, as already described with reference to the balloon catheter 2 of Figs. 1 and 2.

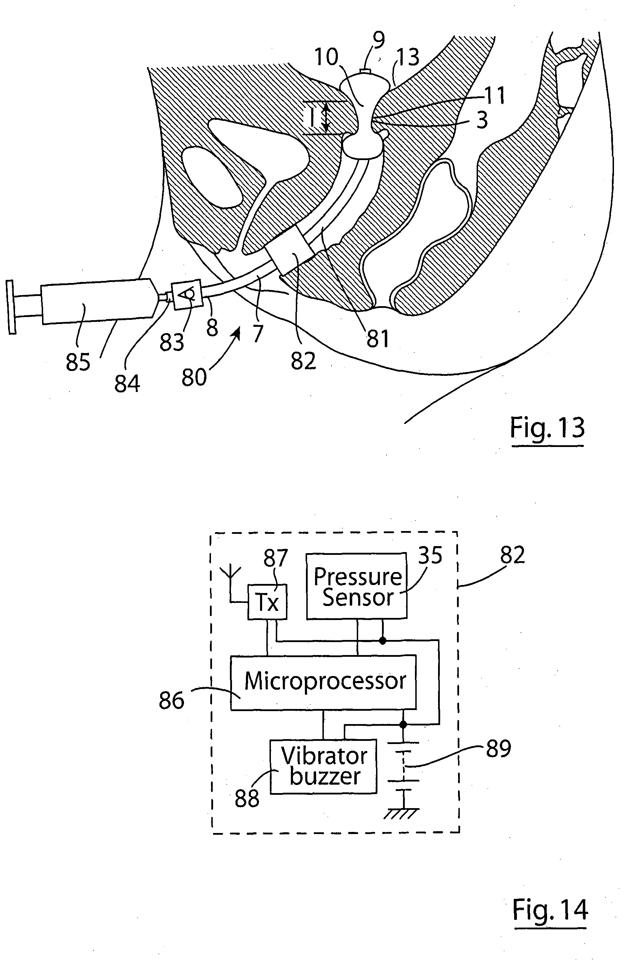

Referring now to Figs. 13 and 14 there is illustrated apparatus according to another embodiment of the invention indicated generally by the reference numeral 80 for monitoring the degree of opening of the cervix 3 in a female human subject, as the cervix 3 ripens prior to the onset of labour in a pregnancy. The apparatus 80 is somewhat similar to the apparatus 1 of Figs. 1 and 2 and similar components are identified by the same reference numerals. The apparatus 80 comprises a balloon catheter 81 which is also according to the invention, and which is somewhat similar to the balloon catheter 2 of Figs. 1 and 2, and similar components are identified by the same reference numerals. In this embodiment of the invention, the apparatus 80 is suitable to be substantially totally self-contained within the cervix 3 and vagina 16 of the female subject.

The balloon catheter 81 comprises a catheter 7 which is substantially similar to the catheter 7 of the balloon catheter 2 of Figs. 1 and 2, but is considerably shorter than the catheter 7 of the balloon catheter 2 of Figs. 1 and 2. In this case, a distal balloon, similar to the distal balloon 12 of the balloon catheter 2 of Figs. 1 and 2 has been omitted, and the balloon catheter 81 comprises only the cervix engaging balloon 10, which is substantially similar to the cervix engaging balloon 10 of the balloon catheter 70 of the embodiment of Figs. 11 and 12. Thus, the cervix engaging balloon 10 of the balloon catheter 81 is located adjacent the distal end 9 of the catheter 7, and is of length *L* which is greater than the length *l* of the opening 11 which extends through the cervix 3 prior to the commencement of ripening thereof.

A housing 82 which is of cylindrical shape is located on the catheter 7 with the catheter 7 extending substantially centrally therethrough and substantially co-axially with the housing 82. The housing 82 is of diameter of approximately 30mm, so that the housing 82 fits comfortably into the vagina 16 of the subject. The catheter 7 is of length from its proximal end 8 to its distal end 9 of approximately 60mm, so that the proximal end 8 of the catheter 7, when the cervix engaging balloon 10 is completely located in the opening 11 extending through the cervix 3, terminates just outside the vagina 16 and adjacent the entrance to the vagina 16.

The proximal end 8 of the catheter 7 terminates in a non-return valve 83 similar to the first non-return valve 27 which is manually releasable, and which communicates with a lumen (not shown) extending through the catheter 7 for inflating the cervix engaging balloon 10. This lumen extending through the catheter 7 is similar to the first lumen 20 which extends through the catheter 7 of the balloon catheter 2 of Figs. 1 and 2. The non-return valve 83 is provided with a luer socket 84 suitable for releasably engaging a luer connector of a medical syringe 85 for inflating the cervix engaging balloon 10 with an inflating medium, namely, air.

Turning now to the housing 82 which is located on the catheter 7 of the balloon catheter 81, the housing 82 houses the pressure sensor 35 which is tapped into the lumen (not shown), similar to the first lumen 20 of the balloon catheter 2 of Figs. 1 and 2, for monitoring the pressure of the inflating medium in the lumen, which is indicative to the pressure of the inflating medium in the cervix engaging balloon 10. The pressure sensor 35 is tapped into the lumen in the portion of the catheter 7 extending through the housing 82. A miniature microprocessor 86 is also located in the housing 82 for reading a signal from the pressure sensor 35, and determining the degree of opening of the cervix 3 during ripening thereof. The microprocessor 86 is programmed in a substantially similar manner to that of the microprocessor 36 of the apparatus of Figs. 1 and 2, and a look-up table, similar to the look-up table 37 is stored in the microprocessor 86. A communications module 87, which is similar to but smaller than the communications module 38 of the apparatus 1, is also located in the housing 82 for transmitting the digital data packages prepared by the microprocessor 86 at the end of the respective predefined time intervals in Bluetooth protocol for reception by a paired Bluetooth enabled smart mobile phone (not shown) which is similar to the smart mobile phone 40 of the apparatus of Figs. 1 and 2, and is configured by the app described with reference to the apparatus of Figs 1 and 2 for receiving the digital data packages from the communications module 87 and for displaying a representation of the current volume of the opening 11 extending through the cervix 3, refreshing the representation in response to the data packages received from the microprocessor 86 at the end of the respective predefined time interval, as already described with reference to the apparatus 1 of Figs. 1 and 2. The representation of the current volume of the opening 11 extending through the cervix 3 which is displayed on the smart mobile phone may be any suitable representation, for example, representations similar to either or both of those illustrated in Figs. 4 and 5.

Additionally, the microprocessor 86 is configured to produce two alert signals, namely, a ripened alert signal indicating that the cervix 3 is fully ripened, and prior to the ripened alert signal, an approaching ripening alert signal indicating that the ripening of the cervix 3 is approaching the fully ripened state, as already described with reference to the apparatus 1 of Figs. 1 and 2. The ripened alert signal and the approaching ripening alert signal are transmitted by the communications module 87 under the control of the microprocessor 86 for reception by the smart mobile phone (not shown). As already described with reference to the apparatus of Figs. 1 and 2, on reception of the alert signals by the smart mobile phone, the smart mobile phone under the control of the app emits the appropriate corresponding ones of the selected audible ring tones as already described with reference to the apparatus 1 of Figs. 1 and 2.

In this embodiment of the invention a means for producing a tactilely perceptible signal and an aurally perceptible signal in response both the ripened alert signal and the approaching ripening alert signal produced by the microprocessor 86 is also provided, and comprises a vibrator/buzzer unit 88. The vibrator/buzzer unit 88 is located in the housing 82, and is secured to the housing 82, so that when activated the vibrator/buzzer unit 88 causes the housing 82 to vibrate. Vibration of the housing 82 induces vibrations in the vagina 16 of the subject. The vibrator/buzzer unit 88 is controlled under the operation of the microprocessor 86, so that the vibrator/buzzer unit 88 is activated in response to the ripened alert signal and the approaching ripening alert signal when the respective signals are produced by the microprocessor 86. The microprocessor 86 is programmed to control the operation of the vibrator/buzzer unit 88 to produce a series of timed spaced apart bursts of vibrations and time spaced apart audible tones in response to the approaching ripening alert signal, and to operate the vibrator/buzzer unit 88 to vibrate continuously and to produce a continuous audible tone in response to the ripened alert signal, in order to alert the subject to the cervix 3 approaching the fully ripened state, and in turn, becoming fully ripened.

In this embodiment of the invention in order to allow a person inflating the cervix engaging balloon 10 with air to monitor the pressure of the inflating medium in the cervix engaging balloon 10 during inflating thereof, the microprocessor 86 in this embodiment of the invention is configured to produce a signal indicative of the pressure of the inflating medium in the cervix engaging balloon 10 continuously during inflating of the balloon 10. The microprocessor 86 operates the communications module 87 to continuously transmit the signal indicative of the pressure of the inflating medium in the cervix engaging balloon 10 during inflating thereof for reception by the smart mobile phone. In this embodiment of the invention the app with which the mobile phone is configured configures the mobile phone to display the numerical value of the pressure of the inflating medium in the cervix engaging balloon 10 during inflating thereof in, for example, a window similar to the window 48 on the display screen 41 illustrated in Fig. 4. Accordingly, a person inflating the cervix engaging balloon 10 by the syringe 85 can read the current pressure of the inflating medium in the cervix engaging balloon 10 from the smart mobile phone.

A battery 89 located in the housing 82 powers the microprocessor 86, the communications module 87, the pressure sensor 35 and the vibrator/buzzer unit 88.

In use, the balloon catheter 81 is inserted through the vagina 16 of the subject until the cervix engaging balloon 10 is located in the opening 11 extending through in the cervix 3. When the cervix engaging balloon 10 is correctly located in the cervix 3, the housing 82 should be located just inside the entrance to the vagina 16, with the non-return valve 83 adjacent the entrance to the vagina 16. The syringe 85 is connected to the luer socket 84 of the non-return valve 83 and the cervix engaging balloon 10 is inflated with the inflating medium, which in this embodiment of the invention is air, to a pressure of approximately 80mmHg. During inflating of the cervix engaging balloon 10, the pressure of the air in the cervix engaging balloon 10 is read from the smart mobile phone. Alternatively, an additional pressure gauge may be provided between the syringe 85 and the non-return valve 83 for monitoring the pressure of the air in the cervix engaging balloon 10 during inflating thereof. When the cervix engaging balloon 10 has been inflated with the inflating air to the pressure of approximately 80mmHg, the syringe 85 is disconnected from the non-return valve 83 which retains the air which has been delivered into the cervix engaging balloon 10 therein.

With the syringe 85 removed from the non-return valve 83, the subject can carry on her normal business with the microprocessor 86 reading the signal from the pressure sensor 35 at the predefined time intervals. The microprocessor 86 determines the volume of the opening 11 extending through the cervix 3 at the end of each predefined time interval, and prepares the digital data package for transmission by the communications module 87 to the smart mobile phone. The subject can then monitor the progress of the ripening of her cervix from her smart mobile phone.

On the microprocessor 86 determining from the signal read from the pressure sensor 35 and the look-up table stored in the microprocessor 86 that the cervix 3 is approaching the fully ripened state, the microprocessor 86 operates the communications module 87 to transmit the approaching ripening alert signal for reception by the smart mobile phone, and the microprocessor 86 also operates the vibrator/buzzer unit 88 to commence intermittent vibrating and to commence production of an intermittent audible tone. On reception by the smart mobile phone of the approaching ripening alert signal, the smart mobile phone activates the appropriate selected one of the ring tones. On the microprocessor 86 determining from the signal read from the pressure sensor 35 and the look-up table stored in the microprocessor 86 that the cervix 3 is in the fully ripened state, the microprocessor 86 operates the communications module 87 to transmit the ripened alert signal for reception by the smart mobile phone, and also operates the vibrator/buzzer unit 88 to continuously vibrate and to produce a continuous audible tone. The smart mobile phone on receiving the ripened alert signal activates the appropriate selected one of the ring tones. Thus, the subject is alerted to both the approach of the cervix being in the fully ripened state, and being in the fully ripened state by both the smart mobile phone and the vibrator/buzzer unit 88.

While the apparatus 80 has been described with the microprocessor 86 configured to communicate via a communications module 87 with a mobile phone, in certain cases it is envisaged that the communications module may be omitted, and the microprocessor would merely be programmed to read the signal from the pressure sensors 35 and determine from the signal read from the pressure sensor 35 and the look-up table, the volume of the opening 11 extending through the cervix 3, and on the volume of the opening 11 extending through the cervix 3 being indicative of the state of the cervix approaching the fully ripened state, the microprocessor 86 would activate the vibrator/buzzer unit 88 to intermittently vibrate and to produce an intermittent audible tone indicating to the subject that fully ripening of the cervix is approaching, and on the volume of the opening extending through the cervix 3 being indicative of the cervix being fully ripened, the microprocessor 86 would activate the vibrator/buzzer unit 88 to continuously vibrate and to produce a continuous audible tone.

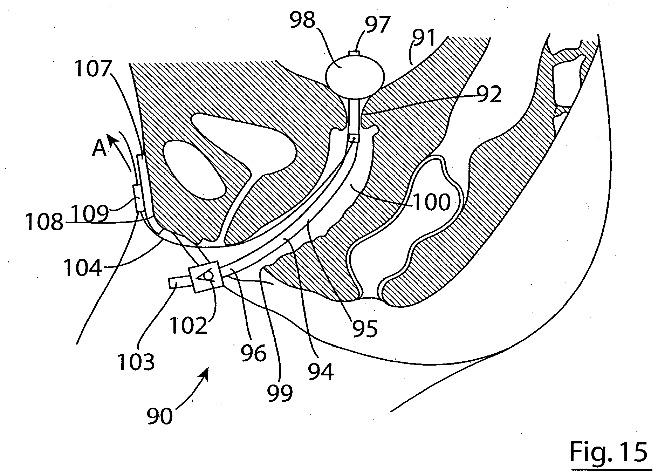

Referring now to Fig. 15 there is illustrated a device which is not according to the invention indicated generally by the reference numeral 90 for stimulating ripening of the cervix of a female human subject, by simulating the effect of the head of a foetus in the uterus 91 of the subject bearing on the cervix 92. The device 90 comprises a balloon catheter 94 comprising an elongated catheter 95 extending between a proximal end 96 a distal end 97. A balloon 98 similar to the distal balloon 12 of the balloon catheter 2 of Figs.1 and 2 is located on the catheter 95 adjacent the distal end 97 with the catheter 95 extending through the balloon 98 to define an annular hollow interior region extending around the catheter 95 similar to the hollow interior region 15 of the balloon 12 of Figs. 1 and 2. The balloon 98 when inflated is of spherical shape, and typically when inflated inflates to a diameter of approximately 40mm to 50mm.

The catheter 95 is relatively short and is of length such that when the balloon 98 is located in the uterus abutting the cervix 92, the proximal end 96 of the catheter 95 terminates just outside the entrance 99 to the vagina 100 of the subject. The proximal end 96 of the catheter 95 terminates in a valve means which permits flow of inflating medium to the balloon 98 for inflating the balloon 98, and prevents return flow of inflating medium from the balloon 98 in order to retain the balloon 98 inflated. In this embodiment of the invention the valve means comprises a manually releasable non-return valve 102 which permits flow of the inflating medium to the balloon 98 for inflating thereof and prevents return flow of the inflating medium from the balloon 98 unless the non-return valve 102 is manually operated to permit return flow of the inflating medium.

A lumen (not shown) similar to the second lumen 22 of the balloon catheter 2 of Figs. 1 and 2 extends through the catheter 95 from the proximal end 96 and terminates in the catheter within the balloon 98. Ports (not shown) similar to the ports 23 of the balloon catheter 2 of Figs. 1 and 2 communicate the lumen extending through the catheter 95 with the hollow interior region of the balloon 98 for inflating thereof. The proximal end of the lumen terminates in the non-return valve 102 for communicating the non-return valve 102 with the hollow interior region of the balloon 98.

A luer socket 103 extends from the non-return valve 102 for releasably coupling the non-return valve 102 to a medical syringe (not shown) for inflating the balloon 98. In this embodiment of the invention the balloon 98 may be inflated with any suitable inflating medium either a compressible or an incompressible fluid, for example, air or a saline solution.

An urging means for urging the balloon 98 into firm engagement with the cervix 92 to simulate the pressure of the head of a foetus in the uterus bearing on the cervix, for stimulating ripening of the cervix prior to the onset of labour, in this embodiment of the invention comprises a tensioning means comprising an elongated ligature 104 which is connected to the catheter 95 at a connection point 105 which is downstream of the balloon 98, and at a location in the vagina 100 below the cervix 92 and slightly spaced apart therefrom. The ligature 104 extends from the connection point 105 on the catheter 95 to an anchoring means, which in this embodiment of the invention is provided by a self-adhesive anchor patch 107.

The anchor patch 107 is adapted for releasably bonding to the subject adjacent the crotch area 108, but spaced apart from the vagina 100, and carries an adjusting means, namely, an adjustable ligature gripping element 109 for releasably and adjustably securing the ligature 104 to the anchor patch 107. In this embodiment of the invention the ligature 104 comprises a resilient elastic material, and when the anchor patch 107 is secured to the crotch area 108, and the ligature 104 is secured to the anchor patch 107, the ligature 104 applies a downward force on the balloon 98, which in turn applies a pressure on the cervix 92 to stimulate ripening of the cervix. The ligature gripping element 109 comprises a means for releasably securing the ligature 104 to the anchor patch 107, which in this case comprises a cam lock gripper (not shown) for releasably gripping the ligature 104 and securing the ligature 104 to the anchor patch 107, when the desired tension has been induced in the ligature 104. The ligature 104 is urged in the direction of the arrow A through a bore (not shown) in the gripping element 109 until the desired tension is induced in the ligature 104, and the ligature 104 is then secured in the bore (not shown) of the gripping element 109 by the cam lock gripper. Needless to say, any other securing means be it releasable or non-releasable may be provided instead of a cam lock gripper. For example, the bore of the gripper element may be provided with a friction grip, which could be releasable or non-releasable.

In use, the catheter 90 with the balloon 98 deflated is urged through the vagina 100, and in turn through the cervix 92 with the distal end 97 and the balloon 98 forming the leading end of the balloon catheter 94. When the balloon 98 is located in the uterus 91, a syringe or other suitable device charged with the inflating medium, which preferably, is a saline solution, is attached to the luer socket 103 of the non-return valve 102. The balloon 98 is then inflated by the syringe with the saline solution to its normal diameter of 40mm to 50mm. The syringe is then disconnected from the non-return valve 102 and the ligature 104 which is now extending from the connecting point 105 downwardly through the vagina 100 is secured to the subject by the anchor patch 107 which is secured to the crotch area 108 of the subject. The tension in the ligature 104 is then adjusted by urging the ligature 104 through the bore (not shown) in the gripping element 109 until the desired tension is achieved, and the ligature 104 is then secured in the gripping element 109 by the cam lock gripper. As ripening of the cervix progresses, the balloon 98 is urged downwardly into the cervix 92, and periodically the tension in the ligature 104 is adjusted , for maintaining a desired degree of tension in the ligature 104, and in turn the desired degree of urging force on the balloon 98 against the cervix 92.

While the ligature 104 has been described as being of a resilient elastic material, it is envisaged that in certain cases the ligature may be of a non-resilient and a non-elastic material, and in which case, a tensioning means, for example, a tensioning spring could be located in a housing mounted on the anchor patch, and the spring would be located in the housing, and one end of the spring would be anchored to the housing, and the other end of the spring would be secured to the ligature. Typically, the spring would be a tension spring, however, it is envisaged that the spring could also be a compression spring. It is also envisaged that a control system could be located in the housing attached to the anchor patch, which would control the tension in the ligature to either maintain the tension in the ligature substantially constant during ripening of the cervix, or to either progressively increase or regressively decrease the pressure in the ligature as ripening progresses.

It will of course be appreciated that a pressure sensing means may be provided for monitoring the pressure of the inflating medium in the balloon 98, and a signal processor may be provided for reading signals produced by the pressure sensing means, and for producing a human sensory perceptible signal indicative of the pressure of the inflating medium in the balloon, both during inflating of the balloon and while the balloon is in the uterus or progressing through the cervix.

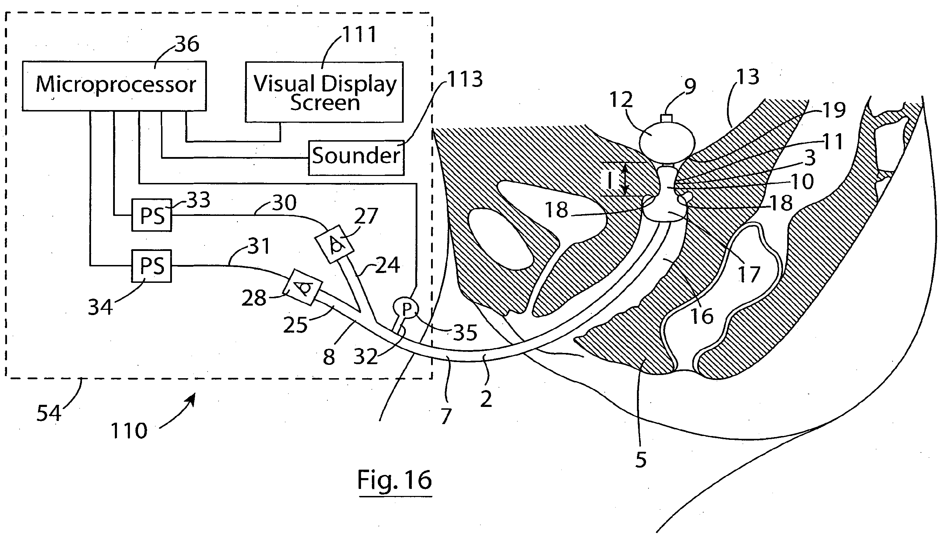

Referring now to Fig. 16 there is illustrated apparatus according to another embodiment of the invention indicated by the reference numeral 110 for monitoring the degree of opening of the cervix in a female human subject as the cervix ripens prior to the onset of labour in a pregnancy. The apparatus 101 is substantially similar to the apparatus 1 described with reference to Figs. 1 to 5, and similar components are identified by the same reference numerals. The main difference between the apparatus 110 and the apparatus 1 is that in this embodiment of the invention a communications module has been omitted, and a visual display means, namely, a visual display screen 111 is mounted on the housing 54 for displaying a representation of the cervix, for example, a representation similar to the representation 42 of the display 39 illustrated in Fig. 4 and/or a representation similar to the representation 49 of Fig. 5 of the degree of ripening of the cervix.

In this embodiment of the invention the visual display screen 111 is mounted on the housing 54 and is hardwired to the microprocessor 36, and is operated under the control of the microprocessor 36 for displaying the display 39 of the representation of the vagina as the vagina ripens.

A means for producing an aurally perceptible signal in this embodiment of the invention comprises a buzzer unit, which in this case comprises a piezo-electric sounder 113, which is located in the housing 54. The piezo-electric sounder 113 is hardwired to the microprocessor 36, and is operated under the control of the microprocessor 36. The microprocessor 36 is programmed to activate the piezo-electric sounder 113 to produce a sequence of time spaced apart audible tones in response to the microprocessor 36 determining that the cervix is approaching the fully ripened state, and the microprocessor 36 is programmed to operate the piezo-electric sounder 113 to produce a continuous audible tone in response to the microprocessor 36 determining that the cervix is in the fully ripened state.

Otherwise the apparatus 110 and its operation is similar to the apparatus 1 described with reference to Figs. 1 to 5.

It is also envisaged that the apparatus 110 may also be provided with a communications module similar to the communications module 38, so that the apparatus 1 would also be suitable for operating in conjunction with a mobile phone as already described with reference to the apparatus 1 described with reference to Figs. 1 to 5.

It is also envisaged that instead of the cervix engaging balloon 10 and the distal balloon 12 of the apparatus 110 of Fig. 16 being inflated by the first and second pumps 33 and 34 under the control of the microprocessor 36, the pumps 33 and 34 may be omitted, and the cervix engaging balloon 10 and the distal balloon 12 would be manually inflated by one or two suitable pumps, for example, medical syringes, of the type described with reference to the apparatus described with reference to Figs. 9 to 14. In which case, the first and second non-return valves would terminate in luer sockets.

It is also envisaged that in the apparatus described with reference to Figs. 1 and 2 and 9 to 12, instead of inflating the cervix engaging and distal balloons with pumps operated under the control of the microprocessor, the balloons could be inflated manually by a medical syringe as described with reference to the apparatus 80 and the balloon catheter 81 of Figs. 13 and 14. It is also envisaged that where the cervix engaging and distal balloons are being inflated under the control of the microprocessor with the same inflating medium, a single pump may be sufficient, and a diverting valve would be provided for diverting the inflating medium from the pump to the appropriate one of the cervix engaging and distal balloons.

It is also envisaged that instead of locating the cervix engaging balloon for monitoring the degree of opening of the cervix on a catheter, in certain cases, it is envisaged that the cervix engaging balloon may be provided without a catheter, and in which case, it would be provided to be mounted on a suitable delivery device for delivering it and placing it in the cervix. In which case, it is envisaged that the cervix engaging balloon may be provided with a housing somewhat similar to the housing 82 of the balloon catheter of Figs. 13 and 14. However, in this case the housing would be attached to the cervix engaging balloon, and the housing would comprise an inflating medium for inflating the cervix engaging balloon. The housing would also comprise a microprocessor which would monitor the pressure of the inflating medium in the cervix engaging balloon, and may or may not be provided with a communications module for communicating with a mobile phone as already described, and the housing may or may not be provided with a vibrator or a vibrator/buzzer unit, which on the microprocessor determining that the cervix is fully ripened, would be activated.

It will also be appreciated that instead of the communications modules of the apparatus of Figs. 1 to 14 being configured to communicate with a mobile phone, the communications modules of the apparatus of Figs. 1 to 14 may be configured to communication with any other suitably enabled and paired smart mobile device, for example, any Bluetooth or otherwise enabled and paired device or any other Bluetooth or otherwise enabled paired smart mobile device configured to communicate wirelessly or otherwise in any other communications protocol. Additionally, the apparatus of any of Figs. 1 to 14 may be provided with a display screen for displaying the representation or representations of the opening extending through the cervix or any other indicia indicative of a characteristic indicative of the opening extending through the cervix, such as, for example, the pressure of the inflating medium in the cervix engaging balloon or the volume of the opening extending through the cervix indicative of the ripening of the cervix.

While the balloons of the balloon catheters have been described as being of polyurethane material, the balloons may be of any suitable material, and indeed, it is envisaged in certain cases that the cervix engaging balloon and the distal balloon may be of different materials.

While the balloons of the balloon catheters have been described as being of specific dimensions, it will be readily apparent to those skilled in the art that the dimensions of the balloons of the balloon catheters may be of any suitable dimensions.

While the balloon catheters have been described for use in monitoring the degree of opening of the cervix prior to the onset of labour in a female human subject, it is envisaged in certain cases that the balloon catheters according to the invention and the apparatus according to the invention may be used in monitoring the degree of opening of the cervix in any female mammal.

While the balloon catheters according to the invention have been described for use with the apparatus according to the invention, it will be readily apparent to those skilled in the art that the balloon catheters according to the invention may be used with a relatively low tech system for inflating the balloons and monitoring the pressure in the cervix engaging balloon as the pressure falls during opening of the cervix. For example, it is envisaged that the cervix engaging and distal balloons, where a distal balloon is provided, may be inflated manually by, for example, a medical syringe, as described with reference to Figs. 13 and 14, and the pressure may be read visually directly from a pressure gauge or a pressure sensor. By comparing the pressure read from the pressure gauge with a printed or other visual look-up table similar to the look-up table of Fig. 3, the degree of opening of the cervix could be readily visually read from the look-up table. Alternatively, it is envisaged that the pressure sensor may comprise a gauge which would be calibrated so that instead of showing the pressure of the inflating medium in the cervix engaging balloon, would show the actual degree of opening of the cervix corresponding to the pressure in the cervix engaging balloon.

It is also envisaged that instead of locating the pressure sensor adjacent the proximal end of the catheter, the pressure sensor may be located within the cervix engaging balloon, or may be located in a recess in the catheter within the cervix engaging balloon whereby the pressure sensor would be subjected to the effect of the pressure of the inflating medium in the cervix engaging balloon. In which case, the pressure sensor may be hardwired through an additional lumen extending through the catheter and connected to either a pressure gauge or a gauge calibrated to directly show the degree of opening of the cervix, or to the microprocessor 35 of the apparatus 1 of Figs. 1 and 2, or the microprocessor 86 of the apparatus of Figs. 13 and 14. Alternatively, in cases where the pressure sensor is located within the cervix engaging balloon or within the catheter within the cervix engaging balloon, the pressure sensor may be coupled directly to a transmitter which would transmit the pressure monitored by the pressure sensor to a pressure gauge or to a gauge calibrated to display the degree of opening of the cervix, or to the microprocessor 35 of the apparatus 1 of Figs. 1 and 2, or to the microprocessor 86 of the apparatus of Figs. 13 and 14.

While the first and second lumens have been described as communicating with the respective cervix engaging and distal balloons through a plurality of communicating ports, it will be readily apparent to those skilled in the art that a single port from each one of the first and second lumens communicating with the corresponding one of the cervix engaging and distal balloons would be sufficient.

While the predefined time interval has been described as being of a specific duration, it will be readily apparent to those skilled in the art that the predefined time interval during which the microprocessor is programmed to read the signal from the pressure sensor may range from as little as 1 second to 30 minutes and even more.

Needless to say, any other signal processor besides a microprocessor may be provided.

It is also envisaged that a tensioning means may be provided for applying a force to the distal balloon or the distal portion of the cervix engaging balloon for urging the distal balloon or the distal portion of the cervix engaging balloon as the case may be in a direction generally outwardly of the vagina for progressively urging the distal balloon or the distal portion of the cervix engaging balloon into and through the opening extending through the cervix as the cervix ripens. Such a tensioning means may be provided by a ligature of, for example, an elastic material or a resilient material, and would typically be connected to the catheter, the distal balloon or the distal portion of the cervix engaging balloon, and would be adapted to be anchored to a suitable part of the body of the subject, for example, to one of the legs of the subject or in the crotch area of the subject adjacent the entrance to the vagina.

It is envisaged that a self-adhesive anchoring pad may be provided which would abut the crotch area of the subject adjacent the vagina and the ligature or other suitable tensioning means would extend through the anchor pad, so that the tension in the ligature or other tensioning means could be selectable and adjustable, as for example, in the device of Fig. 15.

It is also envisaged that the tensioning means may be provided by a spring connected to one of the catheter, the distal balloon or the distal portion of the cervix engaging balloon, and to a suitable anchoring means for anchoring to the subject, and the tension in the tensioning means applied to the distal balloon or the distal portion of the cervix engaging balloon would be selectably adjustable.

Needless to say, any other tensioning means may be provided and any other suitable anchoring means may be provided.

While, in general, the smart mobile device has been described as comprising a smart mobile phone, any other smart mobile device could be configured to communicate with the apparatus of each and every one of the embodiments of the invention described herein. For example, such a smart mobile device may be a tablet device, such as, for example, an iPad (registered Trade Mark), a laptop computer, or any other suitable smart mobile device which could be configured to communicate with the apparatus.

While the signal processing means has been described as comprising a signal processor, any other suitable processing means for processing signals may be used, for example, a programmable logic controller or other such controller or processor.

It is also envisaged that the housing which houses the microprocessor and the other components may be located within the cervix engaging balloon or the distal balloon in the case of the apparatus of Figs. 1 to 14 and 16, and in the case of the balloon located on the distal end of the catheter in the case of the device of Fig. 15. In which case, a suitable channel would be provided for inflating the balloon or balloons, as the case may be.

While the look-up table has been described as correlating reference pressures with reference volumes of the reference cervix, the reference pressures may be correlated with any other corresponding characteristic of the reference cervix, for example, the percentage opening of the reference cervix or any other such suitable corresponding characteristic.

While the correlating means has been described as comprising a correlating table, the correlating means could be provided in any other form, for example, in the form of an electronically stored wave form or the like, whereby the wave form would be a plot of reference pressures against corresponding reference characteristics of the reference cervix.

It is also envisaged that instead of transmitting a data package at the end of each predefined time interval to a paired smart mobile device, a signal indicative of the degree of opening of the cervix could be transmitted, which, for example, may be a numerical value, and the app which configures the smart mobile device could include a correlating means which correlates the numbers, for example, transmitted by the microprocessor through the communications module with corresponding images of the state of a cervix for display on the smart mobile device.

## Claims

1. Apparatus for monitoring the degree of opening of the cervix (3) in a female mammal, the apparatus comprising an inflatable first balloon (10) configured for locating in the cervix (3), a pressure sensing means (35) for monitoring a pressure indicative of the pressure of a compressible inflating medium in the first balloon (10), and a means (36, 37) for determining the degree of opening of the cervix (3) from the pressure of the inflating medium monitored by the pressure sensing means (35), and for producing a signal indicative of the degree of opening of the cervix (3).

2. Apparatus as claimed in Claim 1 **characterised in that** the first balloon (10) is of length to extend through the length (L) of the cervix (3).

3. Apparatus as claimed in Claim 1 or 2 **characterised in that** a retaining means (27) is provided for retaining the inflating medium in the first balloon (10) after it has been inflated in the cervix (3) to a first predefined pressure.

4. Apparatus as claimed in Claim 3 **characterised in that** the retaining means (27) comprises a first valve (27).

5. Apparatus as claimed in Claim 3 or 4 **characterised in that** the retaining means (27) is releasable for deflating the first balloon (10).

6. Apparatus as claimed in any preceding claim **characterised in that** the first balloon (10) is configured so that when the first balloon (10) is located in the cervix (3) and inflated therein to the first predefined pressure prior to commencement of ripening of the cervix (3), the first balloon (10) expands with the opening (11) extending through the cervix, as the opening (11) extending through the cervix (3) expands, and the first balloon (10) remains in contact with the portion of the cervix (3) defining the opening (11) therethrough during ripening of the cervix.

7. Apparatus as claimed in any preceding claim **characterised in that** the means (36, 37) for determining the degree of opening of the cervix (3) from the pressure monitored by the pressure sensing means (35) comprises a signal processing means (36) configured to read a signal from the pressure sensing means (35) and to determine the degree of opening of the cervix in response to the signal read from the pressure sensing means (35).

8. Apparatus as claimed in Claim 7 **characterised in that** a correlating means (37) is provided, the correlating means (37) being configured to correlate a plurality of reference pressure values of an inflating medium in a reference balloon located in a reference cervix with corresponding values of reference degrees of opening of the reference cervix.

9. Apparatus as claimed in Claim 7 or 8 **characterised in that** the signal indicative of the degree of opening of the cervix (3) is configured to be presented as a human sensory perceptible signal.

10. Apparatus as claimed in any of Claims 7 to 9 **characterised in that** the signal processing means (36) is configured to produce at least one of a ripened alert signal in response to the signal read from the pressure sensing means (35) being indicative of the cervix being fully ripened, and an approaching ripening alert signal in response to the signal read from the pressure sensing means (35) being indicative of the cervix approaching the fully ripened state.

11. Apparatus as claimed in any of Claims 7 to 10 **characterised in that** a communicating means (38) is provided for wirelessly transmitting the signals produced by the signal processing means (36).

12. Apparatus as claimed in any preceding claim **characterised in that** the first balloon (10) is located on a catheter (7) of a balloon catheter (2) towards the distal end of the catheter (7), and a second balloon (12) is located on the catheter (7) distally of the first balloon (10) and adjacent thereto, the second balloon (12) being configured for locating in the uterus (13) when the first balloon (10) is located in the cervix (3), and the second balloon (12) is configured to bear on the cervix (3) adjacent the uterus (13).

13. A method for monitoring the degree of opening of a ripening cervix (3) of a female mammal, the method comprising monitoring a pressure indicative of the pressure of a compressible inflating medium in a first balloon (10) inflated to a first predefined pressure located in the cervix, and determining the degree of opening of the cervix (3) from the monitored pressure.

## Patentansprüche

1. Vorrichtung zum Überwachen des Grades der Öffnung des Gebärmutterhalses (3) bei einem weiblichen Säugetier, wobei die Vorrichtung einen aufblasbaren ersten Ballon (10), der zum Anordnen im Gebärmutterhals (3) eingerichtet ist, und ein Drucksensormittel (35) zum Überwachen eines Drucks umfasst, der den Druck eines kompressiblen Aufblasmediums im ersten Ballon (10) anzeigt, und Mittel (36, 37) zum Bestimmen des Grades der Öffnung des Gebärmutterhalses (3) aus dem Druck des Aufblasmediums, der von dem Drucksensormittel (35) überwacht wird, und zum Erzeugen eines Signals, das den Grad der Öffnung des Gebärmutterhalses (3) anzeigt.

2. Vorrichtung nach Anspruch 1, **dadurch gekennzeichnet, dass** der erste Ballon (10) so lang ist, dass er sich durch die Länge (L) des Gebärmutterhalses (3) erstreckt.

3. Vorrichtung nach Anspruch 1 oder 2, **dadurch gekennzeichnet, dass** ein Rückhaltemittel (27) vorgesehen ist, um das Aufblasmedium im ersten Ballon (10) zurückzuhalten, nachdem dieser im Gebärmutterhals (3) auf einen ersten vordefinierten Druck aufgeblasen worden ist.

4. Vorrichtung nach Anspruch 3, **dadurch gekennzeichnet, dass** das Rückhaltemittel (27) ein erstes Ventil (27) umfasst.

5. Vorrichtung nach Anspruch 3 oder 4, **dadurch gekennzeichnet, dass** das Haltemittel (27) zum Entleeren des ersten Ballons (10) lösbar ist.

6. Vorrichtung, nach einem vorhergehenden Ansprüche, **dadurch gekennzeichnet, dass** der erste Ballon (10) so eingerichtet ist, dass, wenn der erste Ballon (10) im Gebärmutterhals (3) angeordnet ist und darin auf den ersten vordefinierten Druck vor Beginn der Reifung des Gebärmutterhalses (3) aufgeblasen wird, der erste Ballon (10) sich mit der Öffnung (11), die sich durch den Gebärmutterhals erstreckt, aufweitet, und der erste Ballon (10) während der Reifung des Gebärmutterhalses in Kontakt mit dem Teil des Gebärmutterhalses (3) bleibt, der die Öffnung (11) durch ihn hindurch definiert.

7. Vorrichtung, nach einem vorhergehenden Ansprüche, **dadurch gekennzeichnet, dass** das Mittel (36, 37) zum Bestimmen des Grades der Öffnung des Gebärmutterhalses (3) aus dem von dem Drucksensormittel (35) überwachten Druck ein Signalverarbeitungsmittel (36) umfasst, das so konfiguriert ist, dass es ein Signal von dem Drucksensormittel (35) liest und den Grad der Öffnung des Gebärmutterhalses als Reaktion auf das von dem Drucksensormittel (35) gelesene Signal bestimmt.

8. Vorrichtung nach Anspruch 7, **dadurch gekennzeichnet, dass** ein Korrelationsmittel (37) vorgesehen ist, wobei das Korrelationsmittel (37) so eingerichtet sind, dass es eine Vielzahl von Referenzdruckwerten eines Aufblasmediums in einem Referenzballon, der sich in einem Referenzhals befindet, mit entsprechenden Werten von Referenzgraden der Öffnung des Referenzhalses korreliert.

9. Vorrichtung nach Anspruch 7 oder 8, **dadurch gekennzeichnet, dass** das Signal, das den Grad der Öffnung des Gebärmutterhalses (3) anzeigt, so eingerichtet ist, dass es als ein vom Menschen sensorisch wahrnehmbares Signal dargestellt wird.

10. Vorrichtung nach einem der Ansprüche 7 bis 9, **dadurch gekennzeichnet, dass** das Signalverarbeitungsmittel (36) so eingerichtet ist, dass es als Reaktion auf das von dem Drucksensormittel (35) gelesenen Signals, das anzeigt, dass der Gebärmutterhals vollständig gereift ist, mindestens ein Reifungswarnsignal und/oder als Reaktion auf das von dem Drucksensormittel (35) gelesenen Signal, das anzeigt, dass der Gebärmutterhals sich dem vollständig gereiften Zustand nähert, ein Reifungswarnsignal erzeugt.

11. Vorrichtung nach einem der Ansprüche 7 bis 10, **dadurch gekennzeichnet, dass** ein Kommunikationsmittel (38) zur drahtlosen Übertragung der von dem Signalverarbeitungsmittel (36) erzeugten Signale vorgesehen ist.

12. Vorrichtung, nach einem vorhergehenden Ansprüche, **dadurch gekennzeichnet, dass** der erste Ballon (10) auf einem Katheter (7) eines Ballonkatheters (2) in Richtung des distalen Endes des Katheters (7) angeordnet ist, und ein zweiter Ballon (12) auf dem Katheter (7) distal vom ersten Ballon (10) und benachbart dazu angeordnet ist, wobei der zweite Ballon (12) zum Anordnen in der Gebärmutter (13) eingerichtet ist, wenn der erste Ballon (10) in der Gebärmutter (13) angeordnet ist, und der zweite Ballon (12) so eingerichtet ist, dass er auf dem Gebärmutterhals (3) neben der Gebärmutter (13) aufliegt.

13. Verfahren zum Überwachen des Öffnungsgrads eines reifenden Gebärmutterhalses (3) eines weiblichen Säugetiers, wobei das Verfahren Überwachen eines Drucks, der den Druck eines kompressiblen Aufblasmediums in einem ersten Ballon (10) anzeigt, der auf einen ersten vordefinierten Druck aufgeblasen wird, der sich im Gebärmutterhals befindet, und Bestimmen des Öffnungsgrads des Gebärmutterhalses (3) aus dem überwachten Druck umfasst.

## Revendications

1. Appareil pour surveiller le degré d'ouverture du col de l'utérus (3) chez un mammifère femelle, l'appareil comprenant un premier ballonnet gonflable (10) configuré pour positionner dans le col de l'utérus (3), un moyen de détection de pression (35) pour surveiller une pression indiquant la pression d'un milieu de gonflage compressible dans le premier ballonnet (10), et un moyen (36, 37) pour déterminer le degré d'ouverture du col de l'utérus (3) à partir de la pression du milieu de gonflage surveillée par le moyen de détection de pression (35), et pour produire un signal indiquant le degré d'ouverture du col de l'utérus (3).

2. Appareil selon la revendication 1, **caractérisé en ce que** le premier ballonnet (10) a une longueur pour s'étendre sur la longueur (L) du col de l'utérus (3).

3. Appareil selon la revendication 1 ou 2, **caractérisé en ce qu'**un moyen de retenue (27) est prévu pour retenir le milieu de gonflage dans le premier ballonnet (10) après qu'il a été gonflé dans le col de l'utérus (3) jusqu'à une première pression prédéfinie.

4. Appareil selon la revendication 3, **caractérisé en ce que** le moyen de retenue (27) comprend une première valve (27).

5. Appareil selon la revendication 3 ou 4, **caractérisé en ce que** le moyen de retenue (27) peut être retiré pour dégonfler le premier ballonnet (10).

6. Appareil selon l'une quelconque des revendications précédentes, **caractérisé en ce que** le premier ballonnet (10) est configuré de sorte que lorsque le premier ballonnet (10) est positionné dans le col de l'utérus (3) et gonflé à l'intérieur de ce dernier jusqu'à la première pression prédéfinie avant le commencement de la maturation du col de l'utérus (3), le premier ballonnet (10) se dilate avec l'ouverture (11) qui s'étend dans le col de l'utérus, au fur et à mesure que l'ouverture (11) qui s'étend dans le col de l'utérus (3) se dilate, et le premier ballonnet (10) reste en contact avec la partie du col de l'utérus (3) définissant l'ouverture (11) dans ce dernier pendant la maturation du col de l'utérus.

7. Appareil selon l'une quelconque des revendications précédentes, **caractérisé en ce que** le moyen (36, 37) pour déterminer le degré d'ouverture du col de l'utérus (3) provenant de la pression surveillée par le moyen de détection de pression (35) comprend un moyen de traitement de signal (36) configuré pour lire un signal provenant du moyen de détection de pression (35) et pour déterminer le degré d'ouverture du col de l'utérus en réponse à la lecture du signal à partir du moyen de détection de pression (35).

8. Appareil selon la revendication 7, **caractérisé en ce qu'**un moyen de corrélation (37) est prévu, le moyen de corrélation (37) étant configuré pour corréler une pluralité de valeurs de pression de référence d'un milieu de gonflage dans un ballonnet de référence positionné dans un col de l'utérus de référence avec des valeurs correspondantes des degrés d'ouverture de référence du col de l'utérus de référence.

9. Appareil selon la revendication 7 ou 8, **caractérisé en ce que** le signal indiquant le degré d'ouverture du col de l'utérus (3) est configuré pour être présenté sous la forme d'un signal sensoriel perceptible par l'être humain.

10. Appareil selon l'une quelconque des revendications 7 à 9, **caractérisé en ce que** le moyen de traitement de signal (36) est configuré pour produire au moins l'un d'un signal d'alerte de maturation en réponse à la lecture de signal du moyen de détection de pression (35) qui indique que le col de l'utérus est complètement mature, et un signal d'alerte de maturation approchant en réponse à la lecture de signal du moyen de détection de pression (35) qui indique que le col de l'utérus s'approche de l'état complètement mature.

11. Appareil selon l'une quelconque des revendications 7 à 10, **caractérisé en ce qu'**un moyen de communication (38) est prévu pour la transmission sans fil des signaux produits par le moyen de traitement de signal (36).

12. Appareil selon l'une quelconque des revendications précédentes, **caractérisé en ce que** le premier ballonnet (10) est positionné sur un cathéter (7) d'un cathéter à ballonnet (2) vers l'extrémité distale du cathéter (7), et un second ballonnet (12) est positionné sur le cathéter (7) de manière distale par rapport au premier ballonnet (10) et adjacent à ce dernier, le second ballonnet (12) étant configuré pour être positionné dans l'utérus (13) lorsque le premier ballonnet (10) est positionné dans le col de l'utérus (3) et le second ballonnet (12) est configuré pour s'appuyer sur le col de l'utérus (3) adjacent à l'utérus (13).

13. Procédé pour surveiller le degré d'ouverture de la maturation du col de l'utérus (3) d'un mammifère femelle, le procédé comprenant l'étape pour surveiller une pression indiquant la pression d'un milieu de gonflage compressible dans un premier ballonnet (10) gonflé à une première pression prédéfinie positionné dans le col de l'utérus, et l'étape pour déterminer le degré d'ouverture du col de l'utérus (3) à partir de la pression surveillée.
